Europäisches Patentamt
European Patent Office
Office européen des brevets

(19) (11) Publication number: 0 257 114 A1

# (12) EUROPEAN PATENT APPLICATION

(21) Application number: 86111581.4

(22) Date of filing: 21.08.86

(51) Int. Cl.4: C12N 15/00 , C07K 15/06 , C12P 21/00 , C12Q 1/68 , A61K 37/02

(43) Date of publication of application: 02.03.88 Bulletin 88/09

(84) Designated Contracting States: GB

(71) Applicant: Kishimoto, Tadamitsu, Prof.
Division of Cellular Immunology Institute of Molecular and Cellular Biology University Osaka
1-3, Yamadaoka Suita Osaka 565(JP)

(72) Inventor: Kishimoto, Tadamitsu
3-5-31, Nakaro Tondabayashi
Osaka 584(JP)
Inventor: Suemura, Masaki, Dr.
9-5, Himemuro
Ikeda Osaka 563(JP)
Inventor: Kikutani, Hitoshi, Dr.
2-1-26, Esaka
Suita Osaka 564(JP)
Inventor: Barsumian, Edward L., Dr.
3-1-503, Senba-Nishi
Mino Osaka 562(JP)

(74) Representative: Laudien, Dieter et al
Boehringer Ingelheim International GmbH
ZA Patente Postfach 200
D-6507 Ingelheim am Rhein-(DE)

## (54) Human low affinity Fc epsilon-receptor, the isolation and purification thereof.

(57) The present invention is concernd with

a) isolation and purification of the soluble IgE binding factor ($Fc_{\epsilon}R$) secreted or shed by lymphoblastoid cells,

b) partial sequencing the obtained $Fc_{\epsilon}R$-receptor, isolating the thus obtained fragments and determination of the sequences of the thus obtained fragments,

c) preparation of an oligonucleotide encoding to one of the said partial sequences,

d) isolating and identifying expression vehicles containing the gene coding for $Fc_{\epsilon}$-receptor, comprising the steps of:

synthesizing cDNA from a RNA matrix derived from lymphoblastoid cells producing $Fc_{\epsilon}$-receptor mRNA,

incorporating said synthesized cDNA in expression vehicles to form an expression vehicle bank,

hydribizing said incorporated cDNA to identify those expression vehicles which contain a gene coding for $Fc_{\epsilon}$-receptor, with a labeled probe comprising an oligonucleotide common to the gene for $Fc_{\epsilon}$--receptor,

replication and expression of the thus obtained gene, and sequencing the thus obtained gene,

EP 0 257 114 A1

e) determination of the gene coding for the active human low-affinity $Fc_{\epsilon}$-receptor utilising isolated cDNA sequence obtained from the vehicles from operation d),

f) expression of the $Fc_{\epsilon}R$ cDNA in Cos 7 cells and

g) preparing an expression vector containing the DNA sequences coding for a soluble fragment of the $Fc_{\epsilon}$-receptor and expressing said soluble fragments in a microorganism or in mammalian cells.

The water-soluble fragment of the $Fc_{\epsilon}$-receptor is suitable for treatment of allergic reactions.

# Human low affinity $Fc_\epsilon$-receptor, the isolation and purification thereof

This invention is concerned with human low affinity $Fc_\epsilon$-receptor and the water-soluble part thereof starting with amino acids from about 40 to 60 of the whole $Fc_\epsilon$-receptor, their isolation and purification. The prepared polypeptides are suitable for the treatment of local and systhemic IgE-allergic reactions.

$Fc_\epsilon$-receptor was found to be an insoluble membrane protein having the unsual and unexpected characteristic of an N-terminal in the cytoplasm and an extra membrane part at the C-terminal side, contrary to known receptors.

Further, it was reported that many cell types bear on their surface IgE receptors ($Fc_\epsilon R$) The IgE receptors on mast cells and basophils are known to have high affinity to the ligand, whereas the homologous receptors on lymphocytes, monocytes and granulocytes are known to be of low affinity. The function of the high affinity $Fc_\epsilon R$ is known, whereas that of the low affinity receptors on lymphocytes is unclear.

Moreover, it is known from the literature, although the crucial role of IgE is not fully appreciated, that IgE biosynthesis requires the interaction of T and B cells and that the regulation of IgE synthesis has certain peculiarities and involves humoral factors derived from other lymphocytes.

Therefore, the following aspects are objects of the present invention:

a) Isolation and purification of the soluble IgE binding factor ($Fc_\epsilon R$) secreted or shed by lymphoblastoid cells.

b) Partial sequencing the $Fc_\epsilon R$-receptor isolated according to a) by means of hydrolysis, isolating the thus obtained fragments and determination of the sequences of the thus obtained fragments.

c) Preparation of an oligonucleotide encoding to one of the said partial sequences isolated according to b), preferably of the oligonucleotide of formula

$$3'\text{-}TT^{T}_{C}\ ACC\ T\overset{T}{\underset{A}{A}}G\ TT^{A}_{G}\ AA^{A}_{G}\ GT\ \text{-}5'$$

encoding for the amino acid sequence of formula

Lys-Trp-Ile-Asn-Phe-Gln-Arg-Lys.

d) Isolating and identifying expression vehicles containing the gene coding for $Fc_\epsilon$-receptor, comprising the steps of:

synthesizing cDNA from a RNA matrix derived from lymphoblastoid cells producing $Fc_\epsilon$-receptor mRNA,

incorporating said synthesized cDNA in expression vehicles to form an expression vehicle bank,

hydribizing said incorporated cDNA to identify those expression vehicles which contain a gene coding for $Fc_\epsilon$-receptor, with a labeled probe comprising an oligonucleotide obtained according to c) common to the gene for $Fc_\epsilon$-receptor,

replication and expression of the thus obtained gene, and sequencing the thus obtained gene.

e) Determination of the gene coding for the active human low-affinity $Fc_\epsilon$-receptor utilising isolated cDNA sequence obtained from the vehicles from operation d) in the sequencing methods of Schreier and Cortese (J. Med. Biol. 129, 169-172 (1979)),

f) Determination that the $Fc_\epsilon R$ cDNA can be expressed in Cos 7 cells and in a vehicle containing a promotor, preferably the sp 6 promotor, and confirming that the expressed product is $Fc_\epsilon R$.

g) Preparing an expression vector containing the DNA sequences coding for a soluble fragment of the $Fc_\epsilon$-receptor and expressing said soluble fragments in a microorganism or in mammalian cells.

a) Isolation and purification of soluble IgE

It was found, that the $Fc_{\epsilon}R$ activity secreted or shed by lymphoblastoid cells, e.g. RPMI8866 cells, as detected by the Elisa method (Figure I) utilizing two different monoclonal antibodies (see European Patent Application No. 86 II0 420.6 of the same applicant, filed on July 29, I986) was higher in the concentrated culture supernatant compared to NP-40 detergent solubilized membrane receptors even though an equal number, e.g., $10^5$ cells/ml were utilized to prepare the $Fc_{\epsilon}R$. Furthermore, when affinitiy purified supernatants were chromatographed on SDS-PAGE under non-reducing conditions and $Fc_{\epsilon}R$ activity eluted from portions of the gel corresponding to defined molecular weight, activity was observed (Figure 2) in the 45-46 kd and 24-25 kd regions. In fact the concentrated culture supernatants contained a higher proportion of activity in the 25 kd region. Therefore serum-free culture supernatants were used as the source of the receptor.

For the sequential immunoaffinity purification of $Fc_{\epsilon}R$ were used immunoaffinity columns which were prepared utilizing I0 mg/ml of purified monoclonal antibody coupled to Sepharose 4B beads (Pharmacia, Piscataway, N.J.) as described by the manufacturer.

The sequential adsorption of 200-250 $\times$ concentrated culture supernatant on BSA-Sepharose, transferrin Sepharose and normal mouse IgG-Sepharose yielded about 70 percent of the original activity without however, improving on the specific activity. As shown in Table I following preadsorption the receptor material was allowed to bind to 3-5-Sepharose column for 4-I6 hours, the total activity of the acetic acid eluate was reduced but the specific activity was increased to 83 units/µg and the purification was I90 fold. Further purification of the receptor by HPLC reverse-phase chromatography on C-4 column using a linear gradient of 0-65 % acetonitril and 0,I % trifluoracetic acid increased the specific activity to I630 units/µg and the receptor was purified 37I0 fold. However, the final recovery was only 33 percent of the original. As seen in Table I, a similar purification scheme was used for detergent-solubilized membrane $Fc_{\epsilon}R$ but the specific activity obtained was consistently lower than that observed with culture supernatants. It is important to note that the choice of elution buffer was critical to the level of recovery, 2.5 percent acetic acid elution with rapid neutralization was important in the final yield of the receptor.

As indicated in Table I immunoaffinity purification of $Fc_{\epsilon}R$ utilizing the specific monoclonal antibodies in the solid phase was not sufficient to obtain pure receptor as measured by a single band on SDS-PAGE. Therefore, freshly eluted $Fc_{\epsilon}R$ was further purified by means of HPLC Reverse Phase Purification. The freshly eluted $Fc_{\epsilon}R$ was loaded preperatively on a C-4 column and chromatographed utilizing a linear gradient of 0-65 percent acetonitril the chromatographic profile is shown on Figure 3, fractions obtained at various retention times were tested by ELISA for $Fc_{\epsilon}R$ activity. It was found that the bulk of the $Fc_{\epsilon}R$ was eluted by the acetonitrile at between 44 and 45 percent concentration. When 0.5 ml samples were collected the $Fc_{\epsilon}R$ activity corresponded to the descending slope of the peak indicated on the Figure 3. The rechromatographic analysis of the active fraction showed a single sharp peak indicating the presence of a homogenous mixture of receptor. The fractions obtained at different retention times were also monitored by SDS-PAGE analysis.

Therefore, the concentrated culture supernatant (crude sup) containing the putative $Fc_{\epsilon}R$ and the material eluted from immunoaffinity and C-4 HPLC column were tested after dialysis and lyophilization on a I0 percent SDS-PAGE as described below. The results indicate the presence of multiple bands in the lane of the crude concentrated sup. A broad band corresponding to 22-24 kd can be seen.

The receptor moiety collected after sequential purification on non-specific and specific immunoaffinity gels still shows multiple bands even though the activity of such eluates is substantially higher than that of crude material. The $Fc_{\epsilon}R$ activity (Figure 4) obtained after C-4 HPLC purification showed a single band corresponding to 25 kd and the material purified in this fashion showed very high activity in the ELISA assay utilizing the monoclonal antibodies. It is important to note that the band of 25 kd corresponds to the minor species of $Fc_{\epsilon}R$ detected by the same monoclonal antibodies after surface iodination and immunoprecipitation of the $Fc_{\epsilon}R$ utilizing the same antibodies, suggesting that the 46 and 25 kd moieties are antigenically identical, and the latter possibly being the soluble by-product of $Fc_{\epsilon}R$.

Although the purification of the IgE binding activity from RPMI8866 cell culture supernatants entailed many steps, it was important to check for the immunological activity of the putative $Fc_{\epsilon}R$ at various stages of purification. In fact equal amounts of HPLC purified $Fc_{\epsilon}R$ were reapplied to specific 3-5-immunoaffinity and non-specific NMIg-Sepharose gels and the activity was monitored in the effluent and eluate of these gels. The results shown in Table 2 indicate that the purified material obviously binds to the specific column and almost all the activity is recovered in the eluate, compared to the non-specific gel where all the disposable activity is found in the effluent and a minor portion in the eluate. It can also be seen that a good portion of the activity is loosely associated to the non-specific gel and lost during washing.

b) Sequencing of the Fc$_{\epsilon}$-receptor

Fc$_{\epsilon}$R prepared after sequential purification of concentrated cell culture supernatant utilizing immunoaffinity gels and C-4 HPLC corresponding to a single band on SDS-PAGE and active in the ELISA assay was subjected to amino acid sequencing. The first attempts indicated that the N-terminal was blocked and therefore two different proteolytic preparations were made utilizing trypsin and lysoendopeptidase. A total of 11 and 12 fragments were obtained with trypsin and lysoendopeptidase treatment respectively. The HPLC profile of the lysoendopeptidase fragmentation shown on figure 5 indicate 12 major peaks corresponding to defined fragments of Fc$_{\epsilon}$R. The following selected number of fragments were obtained:

Gly-Glu-Phe-Ile-Trp-Val-Asp-Gly-Ser-His-Val-Asp-Tyr-Ser-Asn-Trp-Ala-Pro-Gly-Ala-Pro-Thr,

Lys-His-Ala-Ser-His-Thr-Gly-Ser-Trp-Ile-Gly-Leu-Arg-Asn-Leu-Asp-Leu-Lys and

Lys-Trp-Ile-Asn-Phe-Gln-Arg-Lys.

c) Preparation of Oligonucleotides

For example, a mixture of oligonucleotides of formula

$$3'\text{-}TT^{T}_{C}\ ACC\ \overset{T}{\underset{A}{TAG}}\ TT^{A}_{G}\ AA^{A}_{G}\ GT\ \text{-}5'$$

was synthesized by means of an ADI-synthesizer at the 0,2 µMol level according to known methods. The obtained oligonucleotide encoding partially for the amino acid sequence of formula

Lys-Trp-Ile-Asn-Phe-Gln-Arg-Lys

was used as labeled probe to detect the gene for Fc$_{\epsilon}$-receptor in an expression vehicle.

cDNA specific to Fc $_{\epsilon}$R$^{+}$ L cells

Thymidine kinase (TK) deficient L cells, Ltk$^{-}$ cells, were transformed with cellular DNA from RPMI-8866 cells and herpes-simplex virus derived TK gene. After HAT selection, TK positive cells were stained with biotinated anti-Fc$_{\epsilon}$R antibody (8-30) and FITC avidin and sorted by a cell sorter. Fc$_{\epsilon}$R positive L cells were enriched by several cycles of sorting. Two L cell transformed lines, L-V-8-30 and LVI-8-30, which expressed Fc$_{\epsilon}$R, were established from two independent transfection experiments (Fig. 6).

The total RNA were prepared from L-V-8-30 cells by guanidine isothiocyanate/cesium chloride method (see Biochemistry 18, 5294 (1979)). The DNA complementary to mRNA from L-V-8-30 cells was synthesized using the enzyme reverse transcriptase on an oligo (dt) primer. The cDNA was labeled by incorporation of $\alpha$-$^{32}$P-deoxy CTP in this reaction. The $^{32}$P labeled cDNA was mixed and hybridized with 10 fold excess poly-(A)$^{+}$ RNA derived from Ltk$^{-}$ cells. The above mixture was applied on hydroxylapatite column and the unbound single strand cDNA was rehybridized with poly(A)$^{+}$ RNA from Ltk$^{-}$ cells. The single strand cDNA which is specific to Fc$_{\epsilon}$R positive L cell transformant was used as probe to detect the gene for Fc$_{\epsilon}$R in λgt 10 library (Fig. 7).

d) Isolation and identification of expression vehicles containing the gene coding for Fc$_{\epsilon}$-receptor

The exponentially growing RPMI-8866 cells are disrupted in guanidium isothiocyanate solution. The mRNA is isolated by centrifugation on cesium chloride gradient, phenol extraction-and ethanol precipitation. Then, the poly(A)$^{+}$ RNA is isolated by oligo (dt) cellulose chromatography.

The construction of double-stranded cDNA is carried out according to Gubler and Hoffman (Gene 25, 263 (1983)). The poly(A)$^+$ RNA is used as a matrix for the preparation of single-strand cDNA by using reverse transcriptase and an oligo (dt) primer. After treatment with RNase H, the second strand of DNA is synthesized by means of DNA polymerase I. The synthesis of first and second stand of DNA was carried out in a solution containing a mixture of deoxynucleotide triphosphate of adenosine, thymidine, guanosine and cytidine. The double stranded cDNA mixture obtained was then modified by means of the enzyme T4 DNA polymerase to remove any small remaining 3′ overhangs from the first strand cDNA. The EcoRI linkers were added and ligated to the double-stranded cDNA by using the enzyme T4 ligase. The cDNA longer than 1 k bp are fractionated and excess linkers were removed by a Bio-Gel A-50 m column chromatography. The size fractionated cDNA were ligated to EcoRI digested λgt 10 phage vector DNA. The λgt 10 vectors containing the cDNA were packaged in vitro and infected to Escherichia coli strain 0600 hfl.

Among the plaques thus obtained, those which contain sequences specific to $Fc_{\epsilon}R$ were identified by colony hybridization. The test substances used are the radioactively labeled oligonucleotide of formula

$$3'\text{-}TT^{T}_{C}\ ACC\ \overset{T}{\underset{A}{TAG}}\ TT^{A}_{G}\ AA^{A}_{G}\ GT\ \text{-}5'$$

which is specific to a $Fc_{\epsilon}R$ partial sequence and the subtracted labeled cDNA which is specific to $Fc_{\epsilon}R$ positive L cell transformants. One λgt 10 recombinant phage clone which hybridized with both radiolabeled probes was isolated and deposited under number FERM BP-1116 (Fermentation Research Institute, Agency of Industrial Science and Technology, Japan). This λgt 10 clone contained an about 1.7 kd insert. The EcoRI insert from the λgt 10 recombinant DNA clone was labeled by nick translation using $\alpha$-$^{32}$P-dCTP and analysed by Northern hybridization with mRNA from various cells including RPMI-8866, Daudi, CEM, $Fc_{\epsilon}R^+$ L cells and Ltk$^-$ cell. The insert hybridized only with mRNA from Fc $_{\epsilon}$R positive cells such as RPMi-8866 and $Fc_{\epsilon}R^+$ L cells.

In order to determine whether the cDNA can be expressed, the EcoRI insert from the phage clone was ligated to EcoRI digested pDE2 plasmid vector (Fig. 8) containing SV40 early promotor and EcoRI digested pGEM4 (Promega Biotec) containing sp6 promotor (Fig. 9). The used plasmid pDE2-2 is described in the Japanese Patent Publication 1986/88879 from May 07, 1986.

e) Determination of the gene coding for the active human low affinity $Fc_{\epsilon}$-receptor

The complete nucleotide sequence of the EcoRI insert from pGEM4 as shown in table 3 was determined by sequencing of recombinant of DNA utilizing the M13 cloning vector (see J. Mol. Biol. 129, 169-172 (1979)) and confirmed by the procedure of Maxam and Gilbert (see Methods Enzymol. 65, 499), whereby the coding sequence for the human lower affinity $Fc_{\epsilon}$-receptor starts with the ATG-codon (see nucleotides 186-188) and ends with the TGA-codon (see nucleotides 1146-1148).

Therefore, the DNA-sequence and the polypeptide sequence of the human lower affinity $Fc_{\epsilon}$-receptor shows the formula

Met Glu Glu Gly Gln Tyr Ser Glu Ile Glu Glu Leu Pro Arg Arg
ATG GAG GAA GGT CAA TAT TCA GAG ATC GAG GAG CTT CCC AGG AGG
TAC CTC CTT CCA GTT ATA AGT CTC TAG CTC CTC GAA GGG TCC TCC

Arg Cys Cys Arg Arg Gly Thr Gln Ile Val Leu Leu Gly Leu Val
CGG TGT TGC AGG CGT GGG ACT CAG ATC GTG CTG CTG GGG CTG GTG
GCC ACA ACG TCC GCA CCC TGA GTC TAG CAC GAC GAC CCC GAC CAC

Thr Ala Ala Leu Trp Ala Gly Leu Leu Thr Leu Leu Leu Leu Trp
ACC GCC GCT CTG TGG GCT GGG CTG CTG ACT CTG CTT CTC CTG TGG
TGG CGG CGA GAC ACC CGA CCC GAC GAC TGA GAC GAA GAG GAC ACC

His Trp Asp Thr Thr Gln Ser Leu Lys Gln Leu Glu Glu Arg Ala
CAC TGG GAC ACC ACA CAG AGT CTA AAA CAG CTG GAA GAG AGG GCT
GTG ACC CTG TGG TGT GTC TCA GAT TTT GTC GAC CTT CTC TCC CGA

Ala Arg Asn Val Ser Gln Val Ser Lys Asn Leu Glu Ser His His
GCC CGG AAC GTC TCT CAA GTT TCC AAG AAC TTG GAA AGC CAC CAC
CGG GCC TTG CAG AGA GTT CAA AGG TTC TTG AAC CTT TCG GTG GTG

Gly Asp Gln Met Ala Gln Lys Ser Gln Ser Thr Gln Ile Ser Gln
GGT GAC CAG ATG GCG CAG AAA TCC CAG TCC ACG CAG ATT TCA CAG
CCA CTG GTC TAC CGC GTC TTT AGG GTC AGG TGC GTC TAA AGT GTC

Glu Leu Glu Glu Leu Arg Ala Glu Gln Gln Arg Leu Lys Ser Gln
GAA CTG GAG GAA CTT CGA GCT GAA CAG CAG AGA TTG AAA TCT CAG
CTT GAC CTC CTT GAA GCT CGA CTT GTC GTC TCT AAC TTT AGA GTC

Asp Leu Glu Leu Ser Trp Asn Leu Asn Gly Leu Gln Ala Asp Leu
GAC TTG GAG CTG TCC TGG AAC CTG AAC GGG CTT CAA GCA GAT CTG
CTG AAC CTC GAC AGG ACC TTG GAC TTG CCC GAA GTT CGT CTA GAC

```
Ser Ser Phe Lys Ser Gln Glu Leu Asn Glu Arg Asn Glu Ala Ser
AGC AGC TTC AAG TCC CAG GAA TTG AAC GAG AGG AAC GAA GCT TCA
TCG TCG AAG TTC AGG GTC CTT AAC TTG CTC TCC TTG CTT CGA AGT

Asp Leu Leu Glu Arg Leu Arg Glu Glu Val Thr Lys Leu Arg Met
GAT TTG CTG GAA AGA CTC CGG GAG GAG GTG ACA AAG CTA AGG ATG
CTA AAC GAC CTT TCT GAG GCC CTC CTC CAC TGT TTC GAT TCC TAC

Glu Leu Gln Val Ser Ser Gly Phe Val Cys Asn Thr Cys Pro Glu
GAG TTG CAG GTG TCC AGC GGC TTT GTG TGC AAC ACG TGC CCT GAA
CTC AAC GTC CAC AGG TCG CCG AAA CAC ACG TTG TGC ACG GGA CTT

Lys Trp Ile Asn Phe Gln Arg Lys Cys Tyr Tyr Phe Gly Lys Gly
AAG TGG ATC AAT TTC CAA CGG AAG TGC TAC TAC TTC GGC AAG GGC
TTC ACC TAG TTA AAG GTT GCC TTC ACG ATG ATG AAG CCG TTC CCG

Thr Lys Gln Trp Val His Ala Arg Tyr Ala Cys Asp Asp Met Glu
ACC AAG CAG TGG GTC CAC GCC CGG TAT GCC TGT GAC GAC ATG GAA
TGG TTC GTC ACC CAG GTG CGG GCC ATA CGG ACA CTG CTG TAC CTT

Gly Gln Leu Val Ser Ile His Ser Pro Glu Glu Gln Asp Phe Leu
GGG CAG CTG GTC AGC ATC CAC AGC CCG GAG GAG CAG GAC TTC CTG
CCC GTC GAC CAG TCG TAG GTG TCG GGC CTC CTC GTC CTG AAG GAC

Thr Lys His Ala Ser His Thr Gly Ser Trp Ile Gly Leu Arg Asn
ACC AAG CAT GCC AGC CAC ACC GGC TCC TGG ATT GGC CTT CGG AAC
TGG TTC GTA CGG TCG GTG TGG CCG AGG ACC TAA CCG GAA GCC TTG

Leu Asp Leu Lys Gly Glu Phe Ile Trp Val Asp Gly Ser His Val
TTG GAC CTG AAG GGA GAG TTT ATC TGG GTG GAT GGG AGC CAT GTG
AAC CTG GAC TTC CCT CTC AAA TAG ACC CAC CTA CCC TCG GTA CAC

Asp Tyr Ser Asn Trp Ala Pro Gly Glu Pro Thr Ser Arg Ser Gln
GAC TAC AGC AAC TGG GCT CCA GGG GAG CCC ACC AGC CGG AGC CAG
CTG ATG TCG TTG ACC CGA GGT CCC CTC GGG TGG TCG GCC TCG GTC

Gly Glu Asp Cys Val Met Met Arg Gly Ser Gly Arg Trp Asn Asp
GGC GAG GAC TGC GTG ATG ATG CGG GGC TCC GGT CGC TGG AAC GAC
CCG CTC CTG ACG CAC TAC TAC GCC CCG AGG CCA GCG ACC TTG CTG

Ala Phe Cys Asp Arg Lys Leu Gly Ala Trp Val Cys Asp Arg Leu
GCC TTC TGC GAC CGT AAG CTG GGC GCC TGG GTG TGC GAC CGG CTG
CGG AAG ACG CTG GCA TTC GAC CCG CGG ACC CAC ACG CTG GCC GAC

Ala Thr Cys Thr Pro Pro Ala Ser Glu Gly Ser Ala Glu Ser Met
GCC ACA TGC ACG CCG CCA GCC AGC GAA GGT TCC GCG GAG TCC ATG
CGG TGT ACG TGC GGC GGT CGG TCG CTT CCA AGG CGC CTC AGG TAC

Gly Pro Asp Ser Arg Pro Asp Pro Asp Gly Arg Leu Pro Thr Pro
GGA CCT GAT TCA AGA CCA GAC CCT GAC GGC CGC CTG CCC ACC CCC
CCT GGA CTA AGT TCT GGT CTG GGA CTG CCG GCG GAC GGG TGG GGG

Ser Ala Pro Leu His Ser  *
TCT GCC CCT CTC CAC TCT TGA
AGA CGG GGA GAG GTG AGA ACT
```

f) Expression of $Fc_\epsilon R$ cDNA in Cos 7 cells

The EcoRI insert was recovered from λgt I0 phage DNA clone containing $Fc_\epsilon R$ cDNA by EcoRI digestion. The insert was ligated to EcoRI treated expression plasmid vector, pDE-2 which contained two SV40 early promotors (see Fig. 8). Cos 7 cells were transfected with 2 µg/ml of pDE-2 containing $Fc_\epsilon R$ cDNA by DEAE-cextran method and cultured for 2 days. Then, cells were stained with biotinated anti-$Fc_\epsilon R$ (8-30) or biotinated human IgE, developed with FITC-avidin and analysed by FACS. Cos 7 cells, which were transfected with pDE-2 containing $Fc_\epsilon R$ cDNA, reacted specifically with anti-$Fc_\epsilon R$ and human IgE, which confirmed that the cDNA encoded the $Fc_\epsilon R$ (Fig. 8).

The expression of $Fc_\epsilon R$ cDNA in the vector containing sp6 promotor

The EcoRI insert from λgt I0 clone containing $Fc_\epsilon R$ cDNA was ligated to EcoRI digested pGEM4 plasmid vector (Fig. 9). pGEM4 DNA containing $Fc_\epsilon R$ cDNA (pGEM4-$Fc_\epsilon R$) was digested with the enzyme BamHI and linealized. The linealized pGEM4-$Fc_\epsilon R$ was used for sp6 RNA polymerase directed mRNA synthesis. The synthesized mRNA (5 µg) was injected into a Xenopus oocyte. Oocytes were incubated for 48 hrs and lysed in the lysis buffer containing the detergent, NP40. $Fc_\epsilon R$ activity in the lysate was determined by the ELISA using the monoclonal antibodies, 8-30 and 3-5. The lysate from 30 oocytes showed the activity which correspond to that from the culture supernatant of $I \times I0^5$ RPMI-8866 cells (Fig. 9).

g) Preparing an expression vector containing the DNA-sequences coding for a water soluble fragment of $Fc_\epsilon$-receptor

From the obtained gene for $Fc_\epsilon$-receptor (see table 3) were removed by means of a suitable endonuclease the codons coding for the water-insoluble part of $Fc_\epsilon$-receptor, conveniently the codons for the amino acids from about I to 40 to I to 60, preferably I to 45 to I to 55, most preferably I to 50. When introducing the obtained shortened $Fc_\epsilon$-receptor genes into organisms under condition which lead to high yield thereof, there were obtained the desired water-soluble polypeptides without the above mentioned amino acids. Therefore, the water soluble part of $Fc_\epsilon$-receptor contains the follwing sequences:

```
                         Gln Ser Leu Lys Gln Leu Glu Glu Arg Ala
                         CAG AGT CTA AAA CAG CTG GAA GAG AGG GCT
                         GTC TCA GAT TTT GTC GAC CTT CTC TCC CGA

Ala Arg Asn Val Ser Gln Val Ser Lys Asn Leu Glu Ser His His
GCC CGG AAC GTC TCT CAA GTT TCC AAG AAC TTG GAA AGC CAC CAC
CGG GCC TTG CAG AGA GTT CAA AGG TTC TTG AAC CTT TCG GTG GTG

Gly Asp Gln Met Ala Gln Lys Ser Gln Ser Thr Gln Ile Ser Gln
GGT GAC CAG ATG GCG CAG AAA TCC CAG TCC ACG CAG ATT TCA CAG
CCA CTG GTC TAC CGC GTC TTT AGG GTC AGG TGC GTC TAA AGT GTC

Glu Leu Glu Glu Leu Arg Ala Glu Gln Gln Arg Leu Lys Ser Gln
GAA CTG GAG GAA CTT CGA GCT GAA CAG CAG AGA TTG AAA TCT CAG
CTT GAC CTC CTT GAA GCT CGA CTT GTC GTC TCT AAC TTT AGA GTC

Asp Leu Glu Leu Ser Trp Asn Leu Asn Gly Leu Gln Ala Asp Leu
GAC TTG GAG CTG TCC TGG AAC CTG AAC GGG CTT CAA GCA GAT CTG
CTG AAC CTC GAC AGG ACC TTG GAC TTG CCC GAA GTT CGT CTA GAC

Ser Ser Phe Lys Ser Gln Glu Leu Asn Glu Arg Asn Glu Ala Ser
AGC AGC TTC AAG TCC CAG GAA TTG AAC GAG AGG AAC GAA GCT TCA
TCG TCG AAG TTC AGG GTC CTT AAC TTG CTC TCC TTG CTT CGA AGT

Asp Leu Leu Glu Arg Leu Arg Glu Glu Val Thr Lys Leu Arg Met
GAT TTG CTG GAA AGA CTC CGG GAG GAG GTG ACA AAG CTA AGG ATG
CTA AAC GAC CTT TCT GAG GCC CTC CTC CAC TGT TTC GAT TCC TAC

Glu Leu Gln Val Ser Ser Gly Phe Val Cys Asn Thr Cys Pro Glu
GAG TTG CAG GTG TCC AGC GGC TTT GTG TGC AAC ACG TGC CCT GAA
CTC AAC GTC CAC AGG TCG CCG AAA CAC ACG TTG TGC ACG GGA CTT

Lys Trp Ile Asn Phe Gln Arg Lys Cys Tyr Tyr Phe Gly Lys Gly
AAG TGG ATC AAT TTC CAA CGG AAG TGC TAC TAC TTC GGC AAG GGC
TTC ACC TAG TTA AAG GTT GCC TTC ACG ATG ATG AAG CCG TTC CCG

Thr Lys Gln Trp Val His Ala Arg Tyr Ala Cys Asp Asp Met Glu
ACC AAG CAG TGG GTC CAC GCC CGG TAT GCC TGT GAC GAC ATG GAA
TGG TTC GTC ACC CAG GTG CGG GCC ATA CGG ACA CTG CTG TAC CTT
```

```
Gly Gln Leu Val Ser Ile His Ser Pro Glu Glu Gln Asp Phe Leu
GGG CAG CTG GTC AGC ATC CAC AGC CCG GAG GAG CAG GAC TTC CTG
CCC GTC GAC CAG TCG TAG GTG TCG GGC CTC CTC GTC CTG AAG GAC

Thr Lys His Ala Ser His Thr Gly Ser Trp Ile Gly Leu Arg Asn
ACC AAG CAT GCC AGC CAC ACC GGC TCC TGG ATT GGC CTT CGG AAC
TGG TTC GTA CGG TCG GTG TGG CCG AGG ACC TAA CCG GAA GCC TTG

Leu Asp Leu Lys Gly Glu Phe Ile Trp Val Asp Gly Ser His Val
TTG GAC CTG AAG GGA GAG TTT ATC TGG GTG GAT GGG AGC CAT GTG
AAC CTG GAC TTC CCT CTC AAA TAG ACC CAC CTA CCC TCG GTA CAC

Asp Tyr Ser Asn Trp Ala Pro Gly Glu Pro Thr Ser Arg Ser Gln
GAC TAC AGC AAC TGG GCT CCA GGG GAG CCC ACC AGC CGG AGC CAG
CTG ATG TCG TTG ACC CGA GGT CCC CTC GGG TGG TCG GCC TCG GTC

Gly Glu Asp Cys Val Met Met Arg Gly Ser Gly Arg Trp Asn Asp
GGC GAG GAC TGC GTG ATG ATG CGG GGC TCC GGT CGC TGG AAC GAC
CCG CTC CTG ACG CAC TAC TAC GCC CCG AGG CCA GCG ACC TTG CTG

Ala Phe Cys Asp Arg Lys Leu Gly Ala Trp Val Cys Asp Arg Leu
GCC TTC TGC GAC CGT AAG CTG GGC GCC TGG GTG TGC GAC CGG CTG
CGG AAG ACG CTG GCA TTC GAC CCG CGG ACC CAC ACG CTG GCC GAC

Ala Thr Cys Thr Pro Pro Ala Ser Glu Gly Ser Ala Glu Ser Met
GCC ACA TGC ACG CCG CCA GCC AGC GAA GGT TCC GCG GAG TCC ATG
CGG TGT ACG TGC GGC GGT CGG TCG CTT CCA AGG CGC CTC AGG TAC

Gly Pro Asp Ser Arg Pro Asp Pro Asp Gly Arg Leu Pro Thr Pro
GGA CCT GAT TCA AGA CCA GAC CCT GAC GGC CGC CTG CCC ACC CCC
CCT GGA CTA AGT TCT GGT CTG GGA CTG CCG GCG GAC GGG TGG GGG

Ser Ala Pro Leu His Ser  *
TCT GCC CCT CTC CAC TCT TGA
AGA CGG GGA GAG GTG AGA ACT
```

The Fc$_\epsilon$R gene can be introduced into organisms under conditions which lead to high yields thereof, as mentioned hereinbefore. Useful hosts and vectors are well known to those of skill in the art, and reference is made, for example, to European Patent Publication 0 093 6l9 published November 9, l983.

In general, prokaryotes are preferred for expression. For example, E. coli Kl2 strain 294 (ATCC No. 3l446) is particularly useful. Other microbial strains which may be used include E. coli Xl776 (ATCC No. 3l537). The aforementioned strains, as well as E. coli W3ll0 (F$^-$, lambda$^-$, prototrophic, ATCC No. 27325), bacilli such as Bacillus subtilus, and other enterobacteriaceae such as Salmonella typhimurium or Serratia marcenses, and various Pseudomonas species may be used.

In general, plasmid vectors containing replicon and control sequences which are derived from species compatible with the host cell are used in connection with these hosts. The vector ordinarily carries a replication site, as well as marking sequences which are capable of providing phenotypic selection in transformed cells. For example, E. coli is typically transformed using pBR322, a plasmid derived from an Escherichia coli species (Bolivar, et al., Gene 2: 95 (l977)). pBR322 contains genes for ampicillin and tetracyline resistance and thus provides easy means for identifying transformed cells. The pBR322 plasmid, or other microbial plasmids must also contain, or be modified to contain, promotors which can be used by the microbial organism for expression. Those promotors most commonly used in recombinant DNA construction include the beta-lactamase (penicillinase) and lactose promotor systems (Chang et al., Nature 275, 6l5 (l978); Itakura et al., Science l98, l056 (l977); Goeddel et al., Nature 28l, 544 (l979)) and tryptophan (trp) promotor system (Goeddel et al., Nucleic Acids Res. 8, 4057 (l980); EP-A-0 036 776). While these are the most commonly used, other microbial promotors have been discovered and utilized.

For example, the genetic sequence for $Fc_{\epsilon}R$ can be placed under the control of the leftward promotor of bacteriophage Lambda ($P_L$). This promotor is one of the strongest known promotors which can be controlled. Control is exerted by the lambda repressor, and adjacent restriction sites are known. A temperature sensitive allele of this repressor gene can be placed on the vector that contains the complete $Fc_{\epsilon}R$ se quence. When the temperature is raised to 42°C, the repressor is inactivated, and the promotor will be expressed at its maximum level. The amount of mRNA produced under these conditions should be sufficient to result in a cell which contains about l0 % of its newly synthesized RNA originated from the $P_L$ promotor. In this scheme, it is possible to establish a bank of clones in which a functional $Fc_{\epsilon}R$ sequence is placed adjacent to a ribosome binding sequence, and at varying distances from the lambda $P_L$ promotor. These clones can then be screened and the one giving the highest yield selected.

The expression and translation of the Fc $_{\epsilon}$R sequence can also be placed under control of other regulons which may be "homologous" to the organism in its untransformed state. For example, lactose dependent E. coli chromosomal DNA comprises a lactose or lac operon which mediates lactose digestion by expressing the enzyme beta-galactosidase. The lac control elements may be obtained from bacteriophage lambda plaC5, which is infective for E. coli. The phage's lac operon can be derived by transduction from the same bacterial species. Regulons suitable for use in the process of the invention can be derived from plasmid DNA native to the organism. The lac promoter-operator system can be induced by IPTG.

Other promoter-operator systems or portions thereof can be employed as well. For example, the arabinose operator, Colicine $E_1$ operator, galactose operator, alkaline phosphatase operator, trp operator, xylose A operator, tac promotor, and the like can be used.

The genes can be advantageously expressed in expression plasmid pERl03 (E. Rastl-Dworkin et al., Gene 2l, 237-248 (l983); see also European Patent Application No. 83 ll2 8l2.9, dated December 20, l983 Deposit DSM 2773) This vector contains all regulatory elements which lead to a high expression rate of the cloned gene.

In addition to prokaryotes, eukaryotic microbes, such as yeast cultures may also be used. Saccharomyces cerevisiae is the most commonly used among eukaryotic microorganisms, although a number of other species are commonly available. For expression in Saccharomyces, the plasmid YRp7, for example (Stinchcomb, et al., Nature 282, 39, (l979); Kingsman et al., Gene 7, l4l (l979); Tschumper, et al., Gene l0, l57 (l980)) and plasmid YEpl3 (Bwach et al., Gene 8, l2l-l33 (l979)) are commonly used. The plasmid YRp7 contains the TRPl gene which provides a selection marker for a mutant strain of yeast lacking the ability to grow in tryptophan, for example ATCC No. 44076. The presence of the TRPl lesion as a charactristic of the yeast host cell genome then provides an effective environment for detecting transformation by growth in the absence of tryptophan. Similarly, the plasmid YEpl3 contains the yeast LEU2 gene which can be used for complementation of a LEU2 minus mutant strain.

Suitable promoting sequence in yeast vectors include the 5'-flanking region of the genes for ADH I (Ammerer, G., Methods of Enzymology l0l, l92-20l (l983)), 3-phosphoglycerate kinase (Hitzemann, et al., J. Biol. Chem. 255, 2073 (l980)) or other glycolytic enzymes (Kawasaki and Fraenkel, BBRC l08, ll07-lll2 (l982)), such as enolase, glyceraldehyde-3-phosphate dehydrogenase, hexokinase, pyruvate decarboxylase, phosphofructokinase, glucose-6-phosphate isomerase, phosphoglucose isomerase, and glucokinase. In constructing suitable expression plasmids, the termination sequences associated with these genes are also ligated into the expression vector 3' end of the sequence desired to be expressed, to provide polyadenylation of the mRNA and termination.

Other promotors, which have the additional advantage of transcription controlled by growth conditions are the promotor regions of the genes for alcohol dehydrogenase-2, isocy·tochrome C, acid phosphatase, degradative enzymes associated with nitrogen metabolism, the aforementioned glyceraldehyde-3-phosphate dehydrogenase, and enzymes responsible for maltose and galactose utilization. Promotors which are regulated by the yeast mating type locus, such as the promotors of the genes BARl, MR-alpha-l, STE2, STE3, STE5 can be used for temperature regulated system by using temperature dependent siv mutations (Rhine, Ph. D. Thesis, University of Oregon, Eugene, Oregon (l979), Herskowitz and Oshima in The Molecular Biology of the Yeast Sacharomyces, Part I, l8l-209 (l98l), Cold Spring Harbor Laboratory). These mutations directly influence the expressions of the silent mating type cassettes of yeast, and therefore indirectly the mating type dependent promotors.

Generally, however, any plasmid vector containing a yeast-compatible promotor, origin of replication and termination sequences is suitable.

In addition to microorganisms, cultures of cells derived from multicellular organisms may also be used as hosts. In principle, any such cell culture is workable, whether from vertebrate or invertebrate culture. However, interest has been greatest in vertebrate cells, and propagation of vertebrate cells in culture (tissue culture) has become a routine procedure in recent years (Tissue Culture, Academic Press, Kruse and Patterson, Editors (l973)). Examples of such useful host cell lines are VERO and HeLa cells, Chinese hamster ovary (CHO) cell lines, and WI38, BHK, COS-7 and MDCK cell lines. Expression vectors for such cells ordinarily include (if necessary) an origin of replication, a promotor located in front of the gene to be expressed, along with any necessary ribosome binding sites, RNA splice sites, polyadenylation site, and transcriptional terminator sequences.

For use in mammalian cells, the control functions on the expression vectors are often provided by viral material. For example, commonly used promotors are derived from polyoma, Adenovirus 2, and most frequently Simian Virus 40 (SV40). The early and late promotors of SV40 are particularly useful because both are obtained easily from the virus as a fragment which also contains the SV40 viral origin of replication (Fiers et al., Nature 273 , II3 (I978)). Smaller or larger SV40 fragments may also be used, provided there is included the approximately 250 bp sequence extending from the Hind III site toward the Bgl I site location in the viral origin of replication. Further, it is also possible, and often desirable, to utilize promotor or control sequences normally associated with the desired gene sequence, provided such control sequences are compatible with the host cell systems.

An origin of replication may be provided either by construction of the vector to include an exogenous origin, such as may be derived from SV40 or other viral (e.g., Polyoma, Adeno, VSV, BPV, etc.) source, or may be provided by the host cell chromosomal replication mechanism. If the vector is integrated into the host cell chromosome, the latter is often sufficient.

The prepared human lower affinity $Fc_\epsilon$-receptor prepared according to the invention, preferably the water-soluble part thereof, is suitable for the treatment of local and allergic reactions induced by expression of IgE and may be incorporated in the suitable pharmaceutical compositions such as solutions or sprays.

The following examples, which are not exhaustive, will illustriated in the invention in greater detail:

General Materials and Methods:

The monoclonal anti-$Fc_\epsilon R$ antibodies 3-5 ($\gamma_1$) and 8-30 ($\mu$) were produced by hybridization of P3UI myeloma with spleen cells from Balb/c mice immunized with RPM8866 cells (see European Patent Application No. 86 II0 420.6 of the same Applicant, filed on July 29, I986). The 8-30 antibody recognizes an epitope close to the IgE binding site of $Fc_\epsilon R$ and could block binding of IgE to 8866 lymphoblastoid cells. The 3-5 antibody, recognizes a different epitope on Fc $_\epsilon$R and can not block effectively IgE binding to its receptors. These antibodies precipitate 46 kd and 25 kd polypeptides under reducing and non reducing conditions. The monoclonal antibodies were purified from ascitis by 50 % saturated ammonium sulfate precipitation followed by gel filtration using Sepharose 6B (Pharmacia Fine Chemical, Uppsala, Sweden) for IgM class or ion exchange chromatography using QAE-Sephadex (Pharmacia Fine Chemical) for IgGI. The polyclonal mouse IgG was isolated in the same fashion. The anti-mouse IgM-alkaline phosphatase conjugate was purchased from Tago (Burlingame, CA).

Reverse phase HPLC was carried out by using a Waters HPLC system with Hi-Pore, RP-304 (250 × 4.6 mm) (Bio-Rad) column. The immunoaffinity purified Fc R was applied to the reverse phase column equilibrated with water containing 0,I % trifluoracetic acid and eluted with a linear gradient of acetonitril containing 0,I % trifluoroacetic acid (TFA). A flow rate of 0.5 ml/min and a linear gradient (from 0 % to 65 % for 60 min) of acetonitril was employed. The eluted material was frozen and lyophilized prior to testing for activity in ELISA.

$NaDodSO_4$/PAGE

Crude, immunoaffinity purified and HPLC purified fractions of $Fc_\epsilon R$ were analyzed by electrophoresis on a I % $NaDodSO_4$/ I0 % polyacrylamide gel (Fig. 4) and proteins were determined by silver staining using Daiichikagaku silver stain (Daiichi-Kagaku, Tokyo). To measure $Fc_\epsilon R$ activity, after electrophoresis, the gel was cut into 4 mm slices, minced eluted with lysis buffer overnight at room temperature with shaking, the eluted material was collected after centrifugation and tested for activity in ELISA.

Example I

a) Isolation of crude $Fc_{\epsilon}R$ from culture supernatant

RPMI8866 cells were cultured in RPMII640 medium supplemented with I0 % fetal calf serum and antibiotics (I00 units/ml of penicillin and I00 µg/ml of Streptomycin) at a density of $10^6$ cells/ml and a cell viability of 95-99 % in Spinner bottles. The cells were harvested after I5 min centrifugation at 5,000 rpm washed 3 times with Hank's BSS and cultured at the same density in serum-free medium for 48 hours in Spinner bottles. The culture supernatant was collected and supplemented with phenylmethyl sulfonylfluoride (PMSF) (I mM), 0,02 % sodium azide ($NaN_3$), and aprotenin (200 units/ml). The culture supernatants were stored at 4°C during concentration.

Concentration of 8866 culture sups was carried out utilizing an Amicon filter system with DIAFLO, YMI0. The 200-300 times concentrated material was then centrifuged at 85,000 × G for 40 min at 4°C in order to remove insoluble material, whereby crude $Fc_{\epsilon}R$ preparation was obtained.

b) Isolation of $Fc_{\epsilon}R$ from cell lysates

8866 cells (2 × $10^9$ cells) were washed 4 times with PBS and lysed in I0 ml of lysis buffer (PBS containing 0,5 % Nonindet P-40 (NP-40). ImM PMSF for 45 min on ice with periodic vertexing. An additional I0 ml of lysis buffer was added and the lysis continued for an additional 30 min on ice. The lysate was centrifuged at 37.000 rpm for 45 min at 4°C. The lipid layer was carefully removed and the supernatant collected and stored at -70°C.

Example 2

Immunoaffinity Purification

Culture supernatant concentrate (see Example Ia) equivalent to 5-I0 liters of culture were sequentially adsorbed on BSA-Sepharose, human transferrin-Sepharose and normal mouse Ig (NMIg)-Sepharose gels for one hour each at 4°C with rotation. The effluent collected from the last immunoaffinity gel was then applied to anti-$Fc_{\epsilon}R$ affinity column (3-5-Sepharose). Immunoadsorption was allowed to proceed between 4-I6 hours at 4°C with rotation. The gel was poured into a column, the effluent collected and the gel washed sequentially with I50 ml of buffer A (Tris-HCL, I0 mM, pH 7.5, NaCl, 0.5 M, NP-40, 0.5 %), buffer B (Tris-HCl, I0 mM, pH 7.5, NP.40 0,I %), buffer C (Tris-HCl, I0 mM, pH 7.5) and eluted with 2.5 % acetic acid (v/v) and immediately neutralized in Tris-HCl 2M, pH 8.0. The material was stored at -80°C if not used immediately for further purification utilizing HPLC.

Example 3

Enzyme Linked Immunosorbent Assay (ELISA)

The $Fc_{\epsilon}R$ activity was measured by its ability to bind to the monoclonal antibodies 3-5 and 8-30 and monitored utilizing a double antibody enzyme-linked immunosorbent assay (ELISA). 96 well microtiter plates were initially coated with the monoclonal antibody 3-5 I00 µl/well (I0 µg/ml) in coating buffer ($NaHCO_3$ 0.I M, pH 9.6), and incubated overnight at 4°C. The plates were then washed 4 times with rinse buffer, i.e. Dulbecco's, phosphate buffer pH containing 0,05 % Tween 20, followed by the addition of I00 µl test sample diluted with diluent buffer (Tris-HCl 0.05 M, pH 8.I, $MgCl_2$ ImM, NaCl 0,I5 M, Tween 20 0.05 % (v/v), $NaN_3$ 0.02 %, BSA I %). The microtiter plates were incubated for 2 hours at room temperature, and washed 4 times with rinse buffer, followed by the addition of I00 µl of a pretitrated and diluted goat-anti-mouse IgM-alkaline phosphatase conjugates. The plates were incubated for two hours at room temperature and washed 4 times with rinse buffer. In the final step 200 µl of substrate, p-phenyl phosphate disodium (I mg/ml) in substrate buffer ($NaHCO_3$ 0.05 M, pH 9.8, $MgCl_2$, $6H_20$ I0 mM) was added and the colorimetric reaction measured every 30 min for two hours at 405 and 620 nm.

Example 4

Hydrolysis of $Fc_{\epsilon}$-receptor by means of lysoendopeptidase and Separation of Peptides

The N-terminal of the soluble $Fc_{\epsilon}$-receptor being blocked, the native protein was digested with *Achromobacter lyticus* lysoendopeptidase in 50 mM Tris-HCl buffer, pH 9.5 for 6 hours at 35°C at an enzyme-to-substrate ratio of l:500 (W/W). The lyophilized digests were purified by HPLC.

Separation of Peptides by Reverse Phase HPLC

Separation of peptides was performed by HPLC using a C4 column on a Hitachi 655 liquid chromatograph equipped with a 655-60 gradient processor and a Rheodyne Sample injector with a l00 µl sample loop. The elution of peptides was carried out with a linear gradient of 2-propanol: acetonitril = 7:9, v/v from 0-35 % v/v for l hour in 0.l % trifluoracetic acid. The fractionated peptides were manually collected by monitoring the absorbance at 2l5 nm. The flow rate was l.0 ml/min.

Amino Acid Analysis and Sequence Determination

The amino acid analyses were carried out with a Hitachi 835-5 amino acid analyzer after hydrolysis of the peptide fragments in 5.7 HCl at ll0°C in evacuated, sealed tubes for 22-24 hours. Automated Edman degradation was performed with a 470 A protein sequencer (Applied Biosystems, Inc. CA) using a standard program for sequencing. The phenylthiohydantoin (PTH)-amino acids were determined by reverse phase HPLC with isoorátic elution.

The following amino acid sequences were detected:

Gly-Glu-Phe-Ile-Trp-Val-Asp-Gly-Ser-His-Val-Asp-Tyr-Ser-Asn-Trp-Ala-Pro-Gly-Ala-Pro-Thr,

Lys-His-Ala-Ser-His-Thr-Gly-Ser-Trp-Ile-Gly-Leu-Arg-Asn-Leu-Asp-Leu-Lys and

Lys-Trp-Ile-Asn-Phe-Gln-Arg-Lys.

Example 5

Preparation of the oligonucleotide of formula

$$3'\text{-}TT^{T}_{C}\ ACC\ \overset{T}{\underset{A}{TAG}}\ TT^{A}_{G}\ AA^{A}_{G}\ GT\ \text{-}5'$$

The oligonucleotide was synthesized using an ADI-synthesizer at the 0,2 µMol level.

The resulting oligonucleotide was demethylated with thiophenol/triethylamin/dioxan (l:2:2) at room temperature within 90 minutes and washed with methanol (l0 × l ml of methanol).

After removing the demethylated oligonucleotide from controlled pore glass (CPG) and splitting off the protective group with concentrated ammonia within l hour at room temperature and l6 hours 55°C, the oligonucleotide was dried by means of Speedvac®.

The further purification was carried out over 20 % of acrylamide/8 Mol of urea gel with TBE-buffer (l0,9 g of tris (hydroxymethyl)aminomethane, 5,5 g of boric acid and 9,3 g Ethylenedinitrilo-tetraacetic acid disodium salt in l l).

The subsequent gel-electrophorese (40 cm × 25 cm × 0,l cm) was carried out at 50 watt. The l7-mer band was cut out, eluted with water and desalted on a 0,9 × l3 cm Sephadex G 25 medium column in water.

The fractions containing the l7-mer were pooled and dried.
Yield: 7,7 $OD_{260}$ (~ 285 µg)

Figure 3

Purification of soluble $Fc_{\epsilon}R$. Serum-free culture supernatants of RPMI8866 cells concentrated 200 × on Amicon YNI0. Sequentially preadsorbed on BSA-Sepharose, Transferrin-Sepharose, NMIg-Sepharose, followed by specific immunoaffinity chromatography on 3-5-Sepharose, Eluate applied to C-4 HPLC column and eluted with a linear gradient of acetonitril 0-65 % containing 0,I % trifluoroacetic acid. Fractions containing $Fc_{\epsilon}R$ activity are indicated by hatched lines.

Figure 2

NaDodSO₄/PAGE of immunoaffinity purified soluble $Fc_{\epsilon}R$. Immunoaffinity purified soluble $Fc_{\epsilon}R$ derived from RPMI8866 culture supernatants analyzed by $NaDodSO_4$/PAGE under non-reducing conditions. After electrophoresis strips of 4mm in width were cut, minced and eluted in lysis buffer overnight at room temperature. The $Fc_{\epsilon}R$ activity was assessed by an ELISA method utilizing two specific monoclonal antibodies. The regions of specific molecular weight are indicated, on the

Figure I

$Fc_{\epsilon}R$ activity derived from NP-40 detergent solubilized RPMI8866 cells and serum-free culture supernantants (O———O) culture sup (Δ———∇) cell lysate

Figure 5

Peptide map of lysoendopeptides digest of soluble Fc $_\epsilon$R obtained after extensive preadsorption immunoaffinity chromatography and HPLC.

Table 1: Purification of Fc$_\varepsilon$R from RPMI8866 cells

| Material | Total Protein (µg) | Total Activity (units*) | Specific Activity units/µg | Percent Recovery | Purification |
|---|---|---|---|---|---|
| Cell culture Supernatant | 180.000 | 78.800 | 0.44 | 100 | 1 |
| Concentrated Supernatant (190X) | 172.000 | 75.000 | 0.44 | 95.2 | 1 |
| NMIg Effluent | 132.000 | 55.000 | 0.42 | 70 | 1 |
| 3-5-Seph. Eluate | 441 | 36.800 | 83.4 | 47 | 190 |
| HPLC | 16 | 26.000 | 1.630 | 33 | 3.710 |
| Crude Cell lysate | 117.000 | 6.160 | 0.05 | 100 | 1 |
| NMIg Effluent | 99.000 | 2.475 | 0.02 | 40.2 | 0.47 |
| 3-5-Seph. Eluate | 306 | 3.795 | 12.4 | 61.6 | 236 |
| HPLC-purified | - | 649 | 649 | 10.5 | 12.400 |

* 1 unit is the activity of 1 x $10^5$ cell equivalent of 190X concentrated culture supernatant.

Table 2

| Material | 3-5-Sepharose Eluate | Effluent | NMIg-Sepharose Eluate | Effluent | IgE-Sepharose Eluate | Effluent |
|---|---|---|---|---|---|---|
| HPLC purfied | 2.470 | 4.5 | 85.5 | 1.170 | | |

<u>Table 3</u>

```
                     CTCCT GCTTAAACCT CTGTCTCTGA CGGTCCCTGC      35
                     GAGGA CGAATTTGGA GACAGAGACT GCCAGGGACG

CAATCGCTCT GGTCGACCCC AACACACTAG GAGGACAGAC ACAGGCTCCA      85
GTTAGCGAGA CCAGCTGGGG TTGTGTGATC CTCCTGTCTG TGTCCGAGGT

AACTCCACTA AGTGACCAGA GCTGTGATTG TGCCCGCTGA GTGGACTGCG     135
TTGAGGTGAT TCACTGGTCT CGACACTAAC ACGGGCGACT CACCTGACGC

TTGTCAGGGA GTGAGTGCTC CATCATCGGG AGAATCCAAG CAGGACCGCC     185
AACAGTCCCT CACTCACGAG GTAGTAGCCC TCTTAGGTTC GTCCTGGCGG

Met Glu Glu Gly Gln Tyr Ser Glu Ile Glu Glu Leu Pro Arg Arg
ATG GAG GAA GGT CAA TAT TCA GAG ATC GAG GAG CTT CCC AGG AGG   230
TAC CTC CTT CCA GTT ATA AGT CTC TAG CTC CTC GAA GGG TCC TCC

Arg Cys Cys Arg Arg Gly Thr Gln Ile Val Leu Leu Gly Leu Val
CGG TGT TGC AGG CGT GGG ACT CAG ATC GTG CTG CTG GGG CTG GTG   275
GCC ACA ACG TCC GCA CCC TGA GTC TAG CAC GAC GAC CCC GAC CAC

Thr Ala Ala Leu Trp Ala Gly Leu Leu Thr Leu Leu Leu Leu Trp
ACC GCC GCT CTG TGG GCT GGG CTG CTG ACT CTG CTT CTC CTG TGG   320
TGG CGG CGA GAC ACC CGA CCC GAC GAC TGA GAC GAA GAG GAC ACC

His Trp Asp Thr Thr Gln Ser Leu Lys Gln Leu Glu Glu Arg Ala
CAC TGG GAC ACC ACA CAG AGT CTA AAA CAG CTG GAA GAG AGG GCT   365
GTG ACC CTG TGG TGT GTC TCA GAT TTT GTC GAC CTT CTC TCC CGA

Ala Arg Asn Val Ser Gln Val Ser Lys Asn Leu Glu Ser His His
GCC CGG AAC GTC TCT CAA GTT TCC AAG AAC TTG GAA AGC CAC CAC   410
CGG GCC TTG CAG AGA GTT CAA AGG TTC TTG AAC CTT TCG GTG GTG

Gly Asp Gln Met Ala Gln Lys Ser Gln Ser Thr Gln Ile Ser Gln
GGT GAC CAG ATG GCG CAG AAA TCC CAG TCC ACG CAG ATT TCA CAG   455
CCA CTG GTC TAC CGC GTC TTT AGG GTC AGG TGC GTC TAA AGT GTC

Glu Leu Glu Glu Leu Arg Ala Glu Gln Gln Arg Leu Lys Ser Gln
GAA CTG GAG GAA CTT CGA GCT GAA CAG CAG AGA TTG AAA TCT CAG   500
CTT GAC CTC CTT GAA GCT CGA CTT GTC GTC TCT AAC TTT AGA GTC

Asp Leu Glu Leu Ser Trp Asn Leu Asn Gly Leu Gln Ala Asp Leu
GAC TTG GAG CTG TCC TGG AAC CTG AAC GGG CTT CAA GCA GAT CTG   545
CTG AAC CTC GAC AGG ACC TTG GAC TTG CCC GAA GTT CGT CTA GAC

Ser Ser Phe Lys Ser Gln Glu Leu Asn Glu Arg Asn Glu Ala Ser
AGC AGC TTC AAG TCC CAG GAA TTG AAC GAG AGG AAC GAA GCT TCA   590
TCG TCG AAG TTC AGG GTC CTT AAC TTG CTC TCC TTG CTT CGA AGT

Asp Leu Leu Glu Arg Leu Arg Glu Glu Val Thr Lys Leu Arg Met
GAT TTG CTG GAA AGA CTC CGG GAG GAG GTG ACA AAG CTA AGG ATG   635
CTA AAC GAC CTT TCT GAG GCC CTC CTC CAC TGT TTC GAT TCC TAC
```

```
Glu Leu Gln Val Ser Ser Gly Phe Val Cys Asn Thr Cys Pro Glu
GAG TTG CAG GTG TCC AGC GGC TTT GTG TGC AAC ACG TGC CCT GAA   680
CTC AAC GTC CAC AGG TCG CCG AAA CAC ACG TTG TGC ACG GGA CTT

Lys Trp Ile Asn Phe Gln Arg Lys Cys Tyr Tyr Phe Gly Lys Gly
AAG TGG ATC AAT TTC CAA CGG AAG TGC TAC TAC TTC GGC AAG GGC   725
TTC ACC TAG TTA AAG GTT GCC TTC ACG ATG ATG AAG CCG TTC CCG

Thr Lys Gln Trp Val His Ala Arg Tyr Ala Cys Asp Asp Met Glu
ACC AAG CAG TGG GTC CAC GCC CGG TAT GCC TGT GAC GAC ATG GAA   770
TGG TTC GTC ACC CAG GTG CGG GCC ATA CGG ACA CTG CTG TAC CTT

Gly Gln Leu Val Ser Ile His Ser Pro Glu Glu Gln Asp Phe Leu
GGG CAG CTG GTC AGC ATC CAC AGC CCG GAG GAG CAG GAC TTC CTG   815
CCC GTC GAC CAG TCG TAG GTG TCG GGC CTC CTC GTC CTG AAG GAC

Thr Lys His Ala Ser His Thr Gly Ser Trp Ile Gly Leu Arg Asn
ACC AAG CAT GCC AGC CAC ACC GGC TCC TGG ATT GGC CTT CGG AAC   860
TGG TTC GTA CGG TCG GTG TGG CCG AGG ACC TAA CCG GAA GCC TTG

Leu Asp Leu Lys Gly Glu Phe Ile Trp Val Asp Gly Ser His Val
TTG GAC CTG AAG GGA GAG TTT ATC TGG GTG GAT GGG AGC CAT GTG   905
AAC CTG GAC TTC CCT CTC AAA TAG ACC CAC CTA CCC TCG GTA CAC

Asp Tyr Ser Asn Trp Ala Pro Gly Glu Pro Thr Ser Arg Ser Gln
GAC TAC AGC AAC TGG GCT CCA GGG GAG CCC ACC AGC CGG AGC CAG   950
CTG ATG TCG TTG ACC CGA GGT CCC CTC GGG TGG TCG GCC TCG GTC

Gly Glu Asp Cys Val Met Met Arg Gly Ser Gly Arg Trp Asn Asp
GGC GAG GAC TGC GTG ATG ATG CGG GGC TCC GGT CGC TGG AAC GAC   995
CCG CTC CTG ACG CAC TAC TAC GCC CCG AGG CCA GCG ACC TTG CTG

Ala Phe Cys Asp Arg Lys Leu Gly Ala Trp Val Cys Asp Arg Leu
GCC TTC TGC GAC CGT AAG CTG GGC GCC TGG GTG TGC GAC CGG CTG  1040
CGG AAG ACG CTG GCA TTC GAC CCG CGG ACC CAC ACG CTG GCC GAC

Ala Thr Cys Thr Pro Pro Ala Ser Glu Gly Ser Ala Glu Ser Met
GCC ACA TGC ACG CCG CCA GCC AGC GAA GGT TCC GCG GAG TCC ATG  1085
CGG TGT ACG TGC GGC GGT CGG TCG CTT CCA AGG CGC CTC AGG TAC

Gly Pro Asp Ser Arg Pro Asp Pro Asp Gly Arg Leu Pro Thr Pro
GGA CCT GAT TCA AGA CCA GAC CCT GAC GGC CGC CTG CCC ACC CCC  1130
CCT GGA CTA AGT TCT GGT CTG GGA CTG CCG GCG GAC GGG TGG GGG

Ser Ala Pro Leu His Ser  *
TCT GCC CCT CTC CAC TCT TGA GCATGGATA CAGCCAGGCC CAGAGCAAGA  1180
AGA CGG GGA GAG GTG AGA ACT CGTACCTAT GTCGGTCCGG GTCTCGTTCT

CCCTGAAGAC CCCCAACCAC GGCCTAAAAG CCTCTTTGTG GCTGAAAGGT       1230
GGGACTTCTG GGGGTTGGTG CCGGATTTTC GGAGAAACAC CGACTTTCCA

CCCTGTGACA TTTTCTGCCA CCCAAACGGA GGCAGCTGAC ACATCTCCCG       1280
GGGACACTGT AAAAGACGGT GGGTTTGCCT CCGTCGACTG TGTAGAGGGC

CTCCTCTATG GCCCCTGCCT TCCCAGGAGT ACACCCCAAC AGCACCCTCT       1330
GAGGAGATAC CGGGGACGGA AGGGTCCTCA TGTGGGGTTG TCGTGGGAGA
```

```
CCAGATGGGA GTGCCCCCAA CAGCACCCTC TCCAGATGAG AGTACACCCC     1380
GGTCTACCCT CACGGGGGTT GTCGTGGGAG AGGTCTACTC TCATGTGGGG

AACAGCACCC TCTCCAGATG CAGCCCCATC TCCTCAGCAC CCCAGGACCT     1430
TTGTCGTGGG AGAGGTCTAC GTCGGGGTAG AGGAGTCGTG GGGTCCTGGA

GAGTATCCCC AGCTCAGGTG GTGAGTCCTC CTGTCCAGCC TGCATCAATA     1480
CTCATAGGGG TCGAGTCCAC CACTCAGGAG GACAGGTCGG ACGTAGTTAT

AAATGGGGCA GTGATGGCCT CCCA                                 1504
TTTACCCCGT CACTACCGGA GGGT
```

**Claims**

l. Human lower affinity $Fc_\epsilon$-receptor with a molecular weight of about 25 kd.

2. Human lower affinity $Fc_\epsilon$-receptor as claimed in claim I containing the following partial amino acid sequences:

Gly-Glu-Phe-Ile-Trp-Val-Asp-Gly-Ser-His-Val-Asp-Tyr-Ser-Asn-Trp-Ala-Pro-Gly-Ala-Pro-Thr,

Lys-His-Ala-Ser-His-Thr-Gly-Ser-Trp-Ile-Gly-Leu-Arg-Asn-Leu-Asp-Leu-Lys and

Lys-Trp-Ile-Asn-Phe-Gln-Arg-Lys.

3. Human lower affinity $Fc_\epsilon$-receptor as claimed in claim I containing the following partial amino acid sequence:

Lys-Trp-Ile-Asn-Phe-Gln-Arg-Lys.

4. Recombinant human lower affinity $Fc_\epsilon$-receptor as claimed in claim I essentially free of other proteins of human origin.

5. $Fc_\epsilon$-receptor as claimed in claim 4 unaccompanied by associated native glycosylation.

6. $Fc_\epsilon$-receptor as claimed in claims 4 or 5 produced by expression of a recombinant DNA-sequence of formula

```
ATG GAG GAA GGT CAA TAT TCA GAG ATC GAG GAG CTT CCC AGG AGG
TAC CTC CTT CCA GTT ATA AGT CTC TAG CTC CTC GAA GGG TCC TCC

CGG TGT TGC AGG CGT GGG ACT CAG ATC GTG CTG CTG GGG CTG GTG
GCC ACA ACG TCC GCA CCC TGA GTC TAG CAC GAC GAC CCC GAC CAC
```

ACC GCC GCT CTG TGG GCT GGG CTG CTG ACT CTG CTT CTC CTG TGG
TGG CGG CGA GAC ACC CGA CCC GAC GAC TGA GAC GAA GAG GAC ACC

CAC TGG GAC ACC ACA CAG AGT CTA AAA CAG CTG GAA GAG AGG GCT
GTG ACC CTG TGG TGT GTC TCA GAT TTT GTC GAC CTT CTC TCC CGA

GCC CGG AAC GTC TCT CAA GTT TCC AAG AAC TTG GAA AGC CAC CAC
CGG GCC TTG CAG AGA GTT CAA AGG TTC TTG AAC CTT TCG GTG GTG

GGT GAC CAG ATG GCG CAG AAA TCC CAG TCC ACG CAG ATT TCA CAG
CCA CTG GTC TAC CGC GTC TTT AGG GTC AGG TGC GTC TAA AGT GTC

GAA CTG GAG GAA CTT CGA GCT GAA CAG CAG AGA TTG AAA TCT CAG
CTT GAC CTC CTT GAA GCT CGA CTT GTC GTC TCT AAC TTT AGA GTC

GAC TTG GAG CTG TCC TGG AAC CTG AAC GGG CTT CAA GCA GAT CTG
CTG AAC CTC GAC AGG ACC TTG GAC TTG CCC GAA GTT CGT CTA GAC

AGC AGC TTC AAG TCC CAG GAA TTG AAC GAG AGG AAC GAA GCT TCA
TCG TCG AAG TTC AGG GTC CTT AAC TTG CTC TCC TTG CTT CGA AGT

GAT TTG CTG GAA AGA CTC CGG GAG GAG GTG ACA AAG CTA AGG ATG
CTA AAC GAC CTT TCT GAG GCC CTC CTC CAC TGT TTC GAT TCC TAC

GAG TTG CAG GTG TCC AGC GGC TTT GTG TGC AAC ACG TGC CCT GAA
CTC AAC GTC CAC AGG TCG CCG AAA CAC ACG TTG TGC ACG GGA CTT

AAG TGG ATC AAT TTC CAA CGG AAG TGC TAC TAC TTC GGC AAG GGC
TTC ACC TAG TTA AAG GTT GCC TTC ACG ATG ATG AAG CCG TTC CCG

ACC AAG CAG TGG GTC CAC GCC CGG TAT GCC TGT GAC GAC ATG GAA
TGG TTC GTC ACC CAG GTG CGG GCC ATA CGG ACA CTG CTG TAC CTT

GGG CAG CTG GTC AGC ATC CAC AGC CCG GAG GAG CAG GAC TTC CTG
CCC GTC GAC CAG TCG TAG GTG TCG GGC CTC CTC GTC CTG AAG GAC

ACC AAG CAT GCC AGC CAC ACC GGC TCC TGG ATT GGC CTT CGG AAC
TGG TTC GTA CGG TCG GTG TGG CCG AGG ACC TAA CCG GAA GCC TTG

TTG GAC CTG AAG GGA GAG TTT ATC TGG GTG GAT GGG AGC CAT GTG
AAC CTG GAC TTC CCT CTC AAA TAG ACC CAC CTA CCC TCG GTA CAC

GAC TAC AGC AAC TGG GCT CCA GGG GAG CCC ACC AGC CGG AGC CAG
CTG ATG TCG TTG ACC CGA GGT CCC CTC GGG TGG TCG GCC TCG GTC

GGC GAG GAC TGC GTG ATG ATG CGG GGC TCC GGT CGC TGG AAC GAC
CCG CTC CTG ACG CAC TAC TAC GCC CCG AGG CCA GCG ACC TTG CTG

GCC TTC TGC GAC CGT AAG CTG GGC GCC TGG GTG TGC GAC CGG CTG
CGG AAG ACG CTG GCA TTC GAC CCG CGG ACC CAC ACG CTG GCC GAC

GCC ACA TGC ACG CCG CCA GCC AGC GAA GGT TCC GCG GAG TCC ATG
CGG TGT ACG TGC GGC GGT CGG TCG CTT CCA AGG CGC CTC AGG TAC

GGA CCT GAT TCA AGA CCA GAC CCT GAC GGC CGC CTG CCC ACC CCC
CCT GGA CTA AGT TCT GGT CTG GGA CTG CCG GCG GAC GGG TGG GGG

TCT GCC CCT CTC CAC TCT TGA
AGA CGG GGA GAG GTG AGA ACT

or of a degenerative derivate thereof.

7. Water-soluble part of $Fc_\epsilon$-receptor as claimed in claim 6 produced by expression of a recombinat DNA-sequence starting with the codons from about 40 to 60, preferably from 45 to 55, most preferably from 50, of the DNA-sequence as claimed in claim 6.

8. Water-soluble of $Fc_\epsilon$-receptor as claimed in claim 7 produced by expression of a recombinat DNA-sequence containing the sequence of formula

CAG AGT CTA AAA CAG CTG GAA GAG AGG GCT
GTC TCA GAT TTT GTC GAC CTT CTC TCC CGA

GCC CGG AAC GTC TCT CAA GTT TCC AAG AAC TTG GAA AGC CAC CAC
CGG GCC TTG CAG AGA GTT CAA AGG TTC TTG AAC CTT TCG GTG GTG

GGT GAC CAG ATG GCG CAG AAA TCC CAG TCC ACG CAG ATT TCA CAG
CCA CTG GTC TAC CGC GTC TTT AGG GTC AGG TGC GTC TAA AGT GTC

GAA CTG GAG GAA CTT CGA GCT GAA CAG CAG AGA TTG AAA TCT CAG
CTT GAC CTC CTT GAA GCT CGA CTT GTC GTC TCT AAC TTT AGA GTC

GAC TTG GAG CTG TCC TGG AAC CTG AAC GGG CTT CAA GCA GAT CTG
CTG AAC CTC GAC AGG ACC TTG GAC TTG CCC GAA GTT CGT CTA GAC

AGC AGC TTC AAG TCC CAG GAA TTG AAC GAG AGG AAC GAA GCT TCA
TCG TCG AAG TTC AGG GTC CTT AAC TTG CTC TCC TTG CTT CGA AGT

GAT TTG CTG GAA AGA CTC CGG GAG GAG GTG ACA AAG CTA AGG ATG
CTA AAC GAC CTT TCT GAG GCC CTC CTC CAC TGT TTC GAT TCC TAC

GAG TTG CAG GTG TCC AGC GGC TTT GTG TGC AAC ACG TGC CCT GAA
CTC AAC GTC CAC AGG TCG CCG AAA CAC ACG TTG TGC ACG GGA CTT

AAG TGG ATC AAT TTC CAA CGG AAG TGC TAC TAC TTC GGC AAG GGC
TTC ACC TAG TTA AAG GTT GCC TTC ACG ATG ATG AAG CCG TTC CCG

ACC AAG CAG TGG GTC CAC GCC CGG TAT GCC TGT GAC GAC ATG GAA
TGG TTC GTC ACC CAG GTG CGG GCC ATA CGG ACA CTG CTG TAC CTT

```
GGG CAG CTG GTC AGC ATC CAC AGC CCG GAG GAG CAG GAC TTC CTG
CCC GTC GAC CAG TCG TAG GTG TCG GGC CTC CTC GTC CTG AAG GAC

ACC AAG CAT GCC AGC CAC ACC GGC TCC TGG ATT GGC CTT CGG AAC
TGG TTC GTA CGG TCG GTG TGG CCG AGG ACC TAA CCG GAA GCC TTG

TTG GAC CTG AAG GGA GAG TTT ATC TGG GTG GAT GGG AGC CAT GTG
AAC CTG GAC TTC CCT CTC AAA TAG ACC CAC CTA CCC TCG GTA CAC

GAC TAC AGC AAC TGG GCT CCA GGG GAG CCC ACC AGC CGG AGC CAG
CTG ATG TCG TTG ACC CGA GGT CCC CTC GGG TGG TCG GCC TCG GTC

GGC GAG GAC TGC GTG ATG ATG CGG GGC TCC GGT CGC TGG AAC GAC
CCG CTC CTG ACG CAC TAC TAC GCC CCG AGG CCA GCG ACC TTG CTG

GCC TTC TGC GAC CGT AAG CTG GGC GCC TGG GTG TGC GAC CGG CTG
CGG AAG ACG CTG GCA TTC GAC CCG CGG ACC CAC ACG CTG GCC GAC

GCC ACA TGC ACG CCG CCA GCC AGC GAA GGT TCC GCG GAG TCC ATG
CGG TGT ACG TGC GGC GGT CGG TCG CTT CCA AGG CGC CTC AGG TAC

GGA CCT GAT TCA AGA CCA GAC CCT GAC GGC CGC CTG CCC ACC CCC
CCT GGA CTA AGT TCT GGT CTG GGA CTG CCG GCG GAC GGG TGG GGG

TCT GCC CCT CTC CAC TCT TGA
AGA CGG GGA GAG GTG AGA ACT
```

or of a degenerative derivate thereof.

9. $Fc_\epsilon$-receptor as claimed in claim 6, wherein the polypeptide shows the formula

```
Met Glu Glu Gly Gln Tyr Ser Glu Ile Glu Glu Leu Pro Arg Arg
Arg Cys Cys Arg Arg Gly Thr Gln Ile Val Leu Leu Gly Leu Val
Thr Ala Ala Leu Trp Ala Gly Leu Leu Thr Leu Leu Leu Leu Trp
His Trp Asp Thr Thr Gln Ser Leu Lys Gln Leu Glu Glu Arg Ala
Ala Arg Asn Val Ser Gln Val Ser Lys Asn Leu Glu Ser His His
Gly Asp Gln Met Ala Gln Lys Ser Gln Ser Thr Gln Ile Ser Gln
Glu Leu Glu Glu Leu Arg Ala Glu Gln Gln Arg Leu Lys Ser Gln
Asp Leu Glu Leu Ser Trp Asn Leu Asn Gly Leu Gln Ala Asp Leu
Ser Ser Phe Lys Ser Gln Glu Leu Asn Glu Arg Asn Glu Ala Ser
Asp Leu Leu Glu Arg Leu Arg Glu Glu Val Thr Lys Leu Arg Met
Glu Leu Gln Val Ser Ser Gly Phe Val Cys Asn Thr Cys Pro Glu
Lys Trp Ile Asn Phe Gln Arg Lys Cys Tyr Tyr Phe Gly Lys Gly
Thr Lys Gln Trp Val His Ala Arg Tyr Ala Cys Asp Asp Met Glu
Gly Gln Leu Val Ser Ile His Ser Pro Glu Glu Gln Asp Phe Leu
Thr Lys His Ala Ser His Thr Gly Ser Trp Ile Gly Leu Arg Asn
Leu Asp Leu Lys Gly Glu Phe Ile Trp Val Asp Gly Ser His Val
Asp Tyr Ser Asn Trp Ala Pro Gly Glu Pro Thr Ser Arg Ser Gln
Gly Glu Asp Cys Val Met Met Arg Gly Ser Gly Arg Trp Asn Asp
Ala Phe Cys Asp Arg Lys Leu Gly Ala Trp Val Cys Asp Arg Leu
Ala Thr Cys Thr Pro Pro Ala Ser Glu Gly Ser Ala Glu Ser Met
Gly Pro Asp Ser Arg Pro Asp Pro Asp Gly Arg Leu Pro Thr Pro
Ser Ala Pro Leu His Ser  *
```

10. $Fc_\epsilon$-receptor as claimed in claim 7 without the amino acids from about l to 40 to l to 60, preferably from l to 45 to l to 55, most preferably l to 50.

11. $Fc_\epsilon$-receptor as claimed in claim 10, wherein the polypeptide shows the partial formula

```
                        Gln Ser Leu Lys Gln Leu Glu Glu Arg Ala
Ala Arg Asn Val Ser Gln Val Ser Lys Asn Leu Glu Ser His His
Gly Asp Gln Met Ala Gln Lys Ser Gln Ser Thr Gln Ile Ser Gln
Glu Leu Glu Glu Leu Arg Ala Glu Gln Gln Arg Leu Lys Ser Gln
Asp Leu Glu Leu Ser Trp Asn Leu Asn Gly Leu Gln Ala Asp Leu
Ser Ser Phe Lys Ser Gln Glu Leu Asn Glu Arg Asn Glu Ala Ser
Asp Leu Leu Glu Arg Leu Arg Glu Glu Val Thr Lys Leu Arg Met
Glu Leu Gln Val Ser Ser Gly Phe Val Cys Asn Thr Cys Pro Glu
Lys Trp Ile Asn Phe Gln Arg Lys Cys Tyr Tyr Phe Gly Lys Gly
Thr Lys Gln Trp Val His Ala Arg Tyr Ala Cys Asp Asp Met Glu
Gly Gln Leu Val Ser Ile His Ser Pro Glu Glu Gln Asp Phe Leu
Thr Lys His Ala Ser His Thr Gly Ser Trp Ile Gly Leu Arg Asn
Leu Asp Leu Lys Gly Glu Phe Ile Trp Val Asp Gly Ser His Val
Asp Tyr Ser Asn Trp Ala Pro Gly Glu Pro Thr Ser Arg Ser Gln
Gly Glu Asp Cys Val Met Met Arg Gly Ser Gly Arg Trp Asn Asp
Ala Phe Cys Asp Arg Lys Leu Gly Ala Trp Val Cys Asp Arg Leu
Ala Thr Cys Thr Pro Pro Ala Ser Glu Gly Ser Ala Glu Ser Met
Gly Pro Asp Ser Arg Pro Asp Pro Asp Gly Arg Leu Pro Thr Pro
Ser Ala Pro Leu His Ser  *
```

12. A recombinant DNA molecule which contains the genetic information as claimed in claims 4 to 11 or a degenerative derivate thereof.

13. A recombinant DNA molecule as claimed in claim 12 which is a plasmid vector, phage vector or cosmid vector.

14. The plasmid of claim 13 wherein the coding sequence is as claimed in claim 6, 10 or 11.

15. The plasmid of claim 13 deposited in E. coli HB 101 under number FERM BP-1116 containing the DNA-sequence as claimed in claim 6.

16. A recombinant DNA molecule as claimed in claim 13 containing the replicon and control sequences for expression.

17. A recombinant DNA molecule as claimed in claim 16 which is an expression vector suitable for transformation of E. coli.

18. A recombinant DNA molecule as claimed in claim 16 which is a yeast expression vector.

19. A host organism transformed by a vector as claimed in any one of claims 9 to 18.

20. An oligonucleotide encoding for a partial amino acid sequence as claimed in claim 2 or 3.

21. The oligonucleotide of the formula

$$3'\text{-}TT^{T}_{C}\ ACC\ TA\overset{T}{\underset{A}{G}}\ TT^{A}_{G}\ AA^{A}_{G}\ GT\ \text{-}5'$$

encoding for the amino acid sequence as claimed in claim 3.

22. A process for preparing a polypeptide as claimed in any one of claims 4 to 11 which comprises transforming a suitable host organism with an expression vector as claimed in any we of claims 9 to 18 containing a coding sequence for the desired polypeptide at an appropriate site for expression and isolating the desired polypeptide from the resulting transformants.

23. Process for the preparation of human lower affinity $Fc_\epsilon$-receptor as claimed in claim 1 which comprises

culturing B lymphoblastoid cells and separating said $Fc_\epsilon R$ from the supernatant or from the lysed cells by sequential immunoaffinity purification.

24. Process as claimed in claim 23, wherein RPMI8866 cells are used as lymphoblastoid cells.

25. Process as claimed in claims 23 or 24, wherein a solution containing $Fc_\epsilon R$ is sequentially absorbed on BSA-Sepharose, human transferin Sepharose and normal mouse Ig-Sepharose, the effluent is applied to an anti-Fc R-affinity column and the eluent is further purified by means of HPLC.

26. Process as claimed in claim 25, wherein the immunoaffinity column is prepared by coupling a corresponding antibody to Sepharose.

27. Process for the preparation of partial amino acid sequences as claimed in claims 2 or 3 wherein $Fc_\epsilon$-receptor as prepared according to claim 23 is subjected to trypsin or lysoendopeptidase treatment and subsequently separated by means of HPLC.

28. Process as claimed in claim 27, wherein the partial amino acid sequence as claimed in claim 3 is isolated.

29. Process for the preparation of the DNA-sequence as claimed in claim 20 or 2l wherein the oligonucleotide is synthesized by addition of the corresponding nucleotides by means of an oligo synthesizer.

30. A process for identifying expression vehicles containing genes coding for $Fc_\epsilon R$ as claimed in claims 4 to ll, comprising the steps of:

synthesizing cDNA from an RNA matrix derived from lymphoblastoid cells producing $Fc_\epsilon R$ mRNA,

incorporating said synthesized cDNA in expression vehicles to form an expression vehicle bank,

hybridizing said incorporated cDNA to identify those expression vehicles which contain a gene coding for $Fc_\epsilon R$, with a labeled probe comprising an oligonucleotide common to the gene for $Fc_\epsilon R$.

3l. Process as claimed in claim 30, wherein said probe is synthesized from a matrix of RNA containing $Fc_\epsilon R$ sequences and said oligonucleotide is a primer.

32. Process as claimed in claim 30, wherein said oligonucleotide is as claimed in claim 20 or 2l.

33. Process as claimed in claim 30, wherein said lymphoblastoid cell are cells of line RPMI8866.

34. Process for preparing a host organism as claimd in claim l9, wherein a vector as claimed in claims l3 to l8 is transformed with a suitable host.

35. Process for preparing a vector as claimed in claims l3 to l8, wherein a DNA-sequence as claimed in claims 4 to ll is inserted in a suitable vector.

36. Pharmaceutical compositions containing a polypeptide as claimed in claim l to ll.

37. Pharmaceutical compositions as claimed in claim 36 suitable for treatment of local or systhemic IgE-allergic reactions.

Figure 1

FcER activity in cell lysate and sup. of RPMI8866 cells

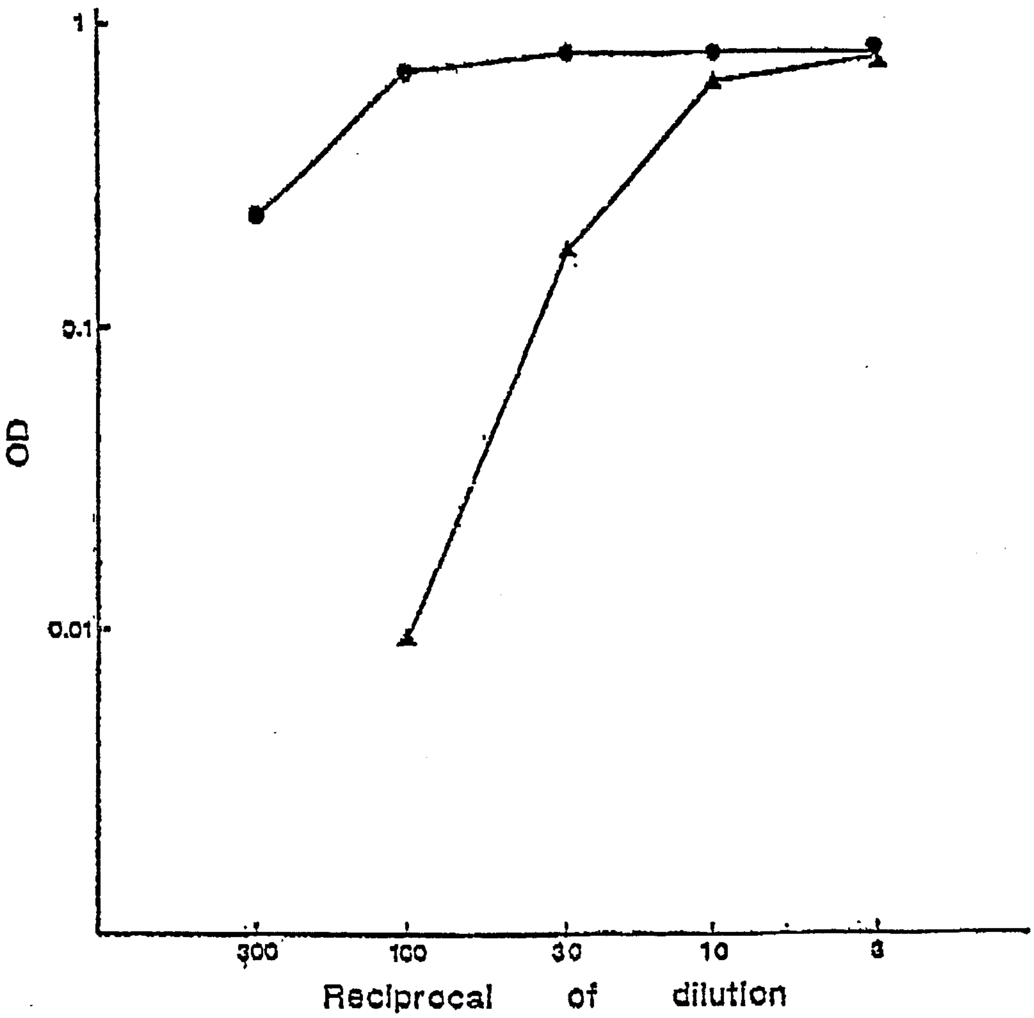

Figure 2

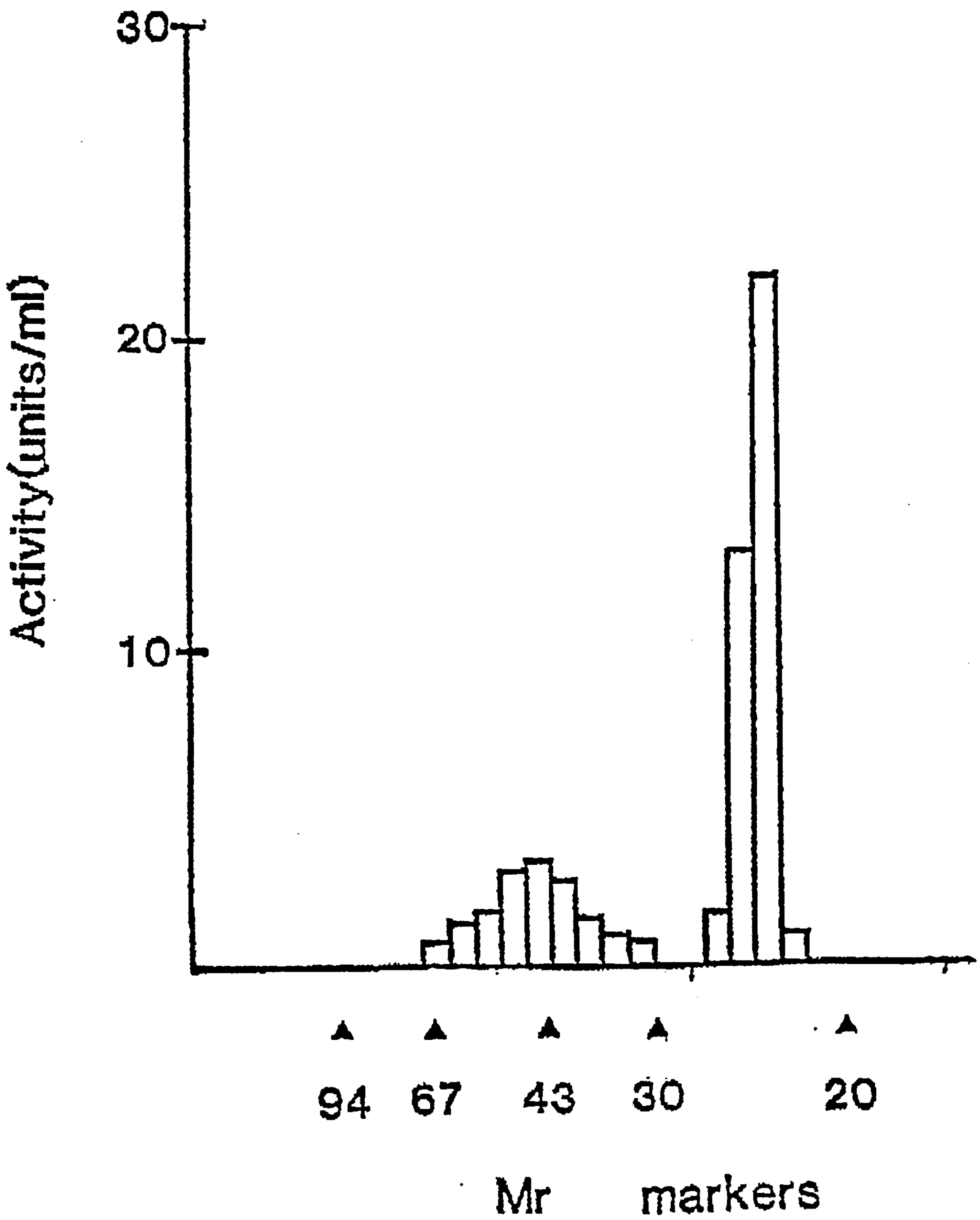

Figura 3

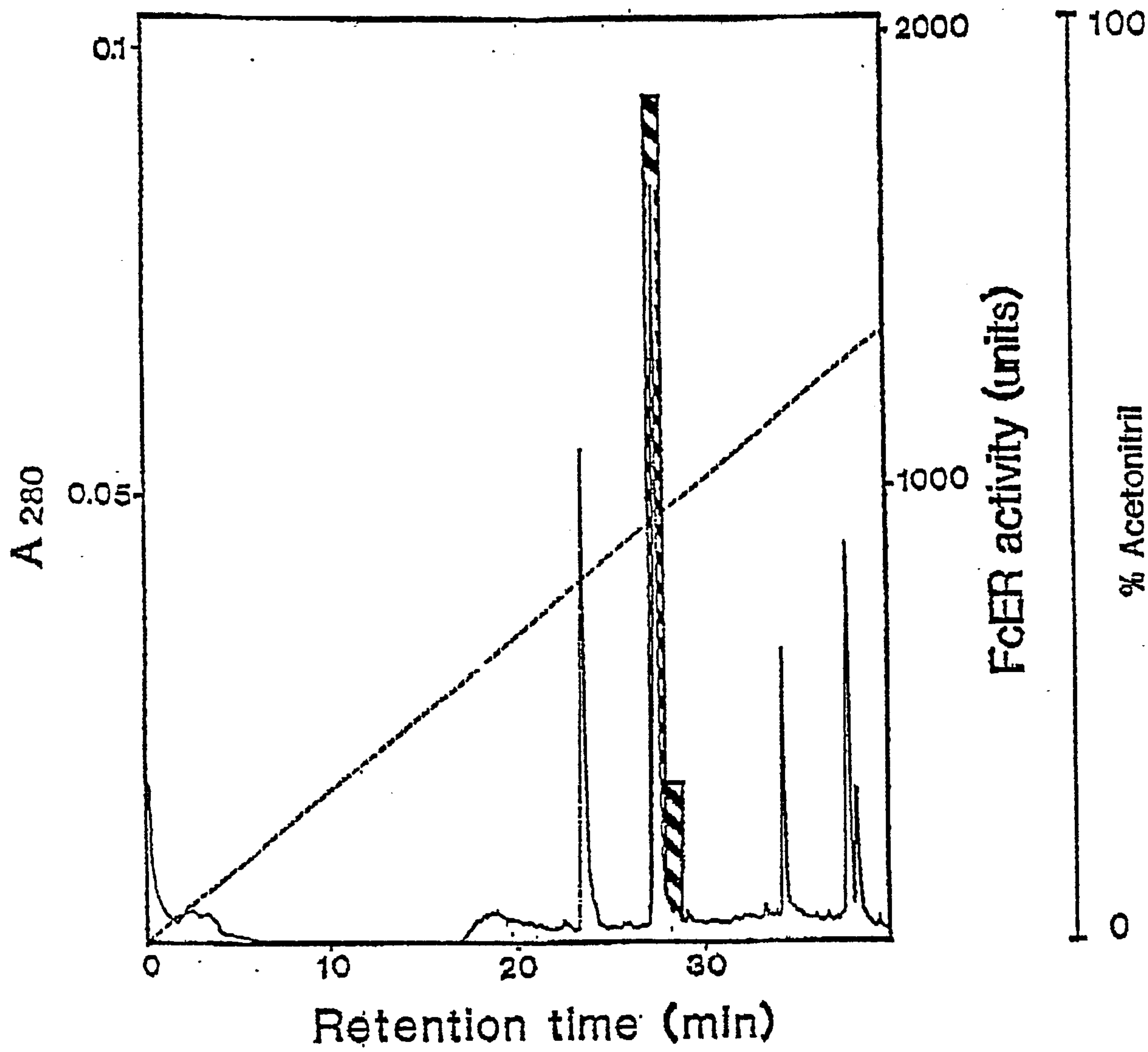
0.1
0.05
A 280
2000
1000
FcER activity (units)
100
0
% Acetonitril
0
10
20
30
Retention time (min)

Fig. 4

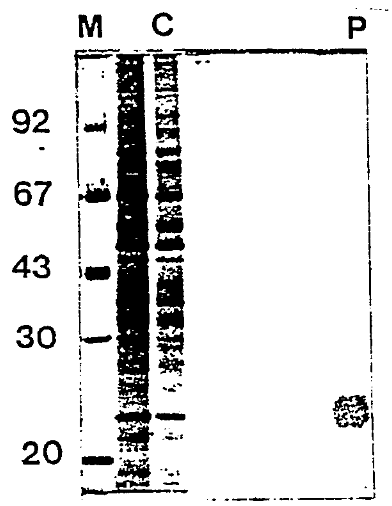

Fig. 5

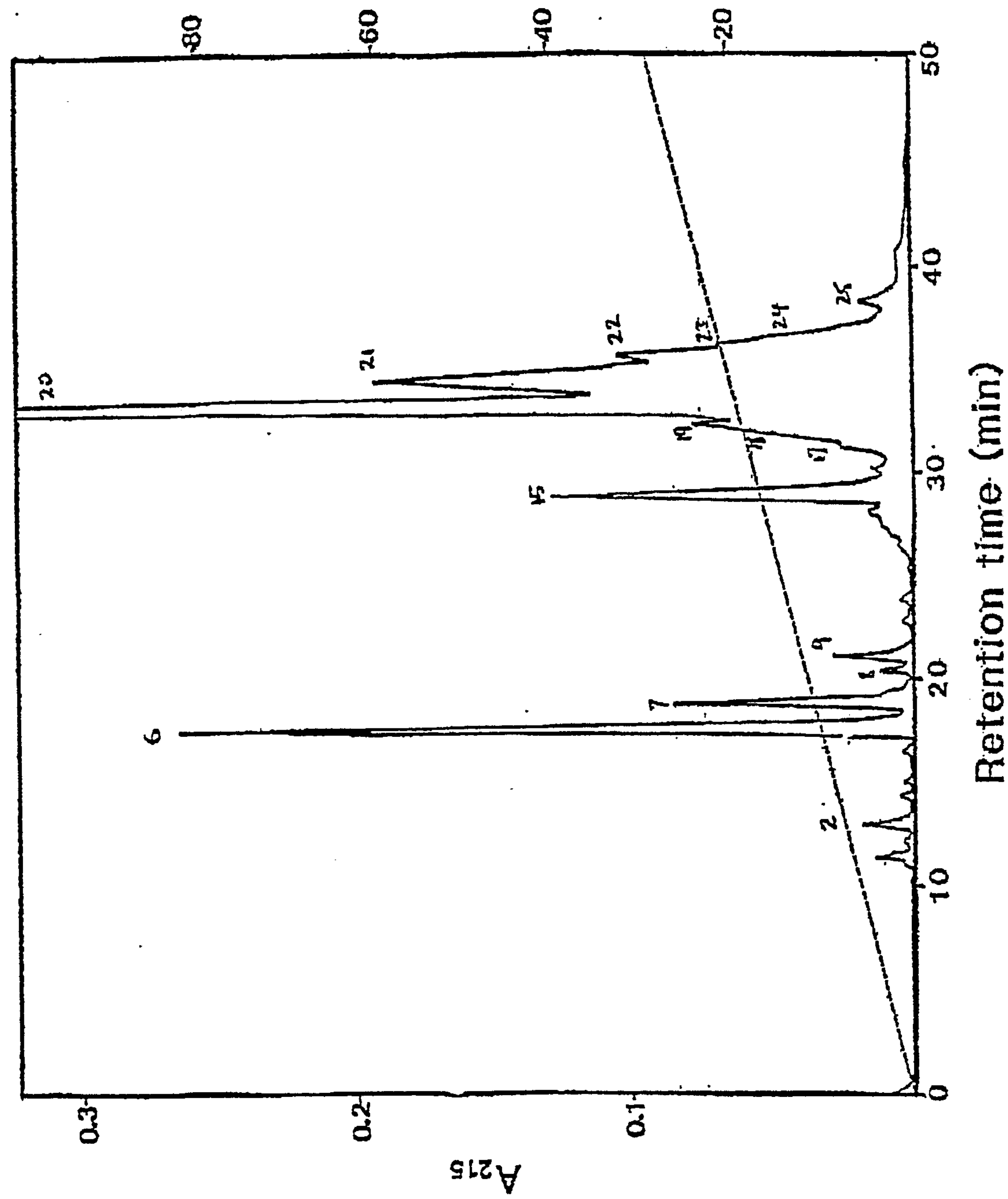

Retention time (min)
$A_{215}$
0
10
20
30
40
50
0.1
0.2
0.3
20
40
60
80
2
6
7
15
20
21
22
23
24
25

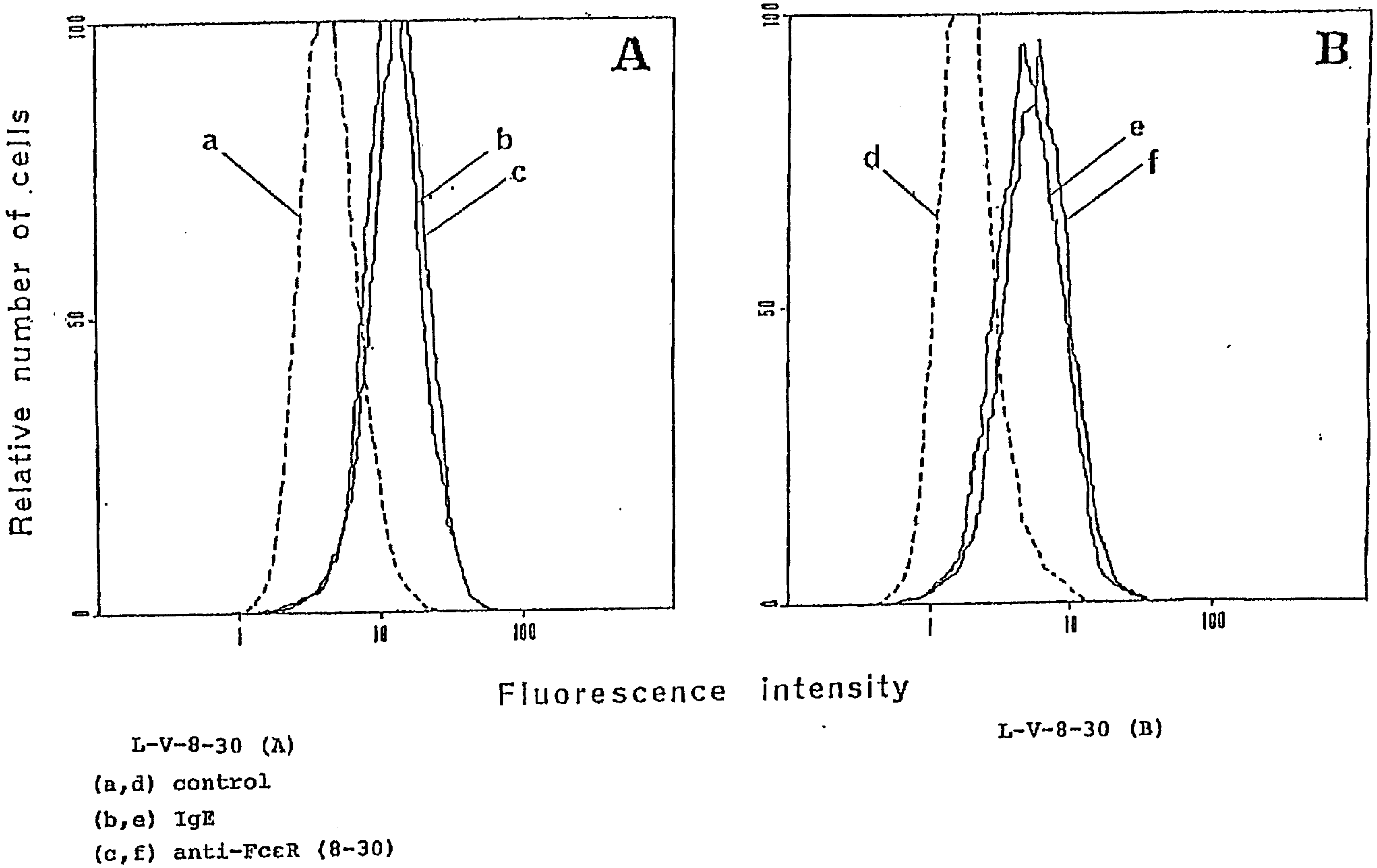
Relative number of cells
100
50
0
A
B
a
b
c
d
e
f
1
10
100
Fluorescence intensity
L-V-8-30 (A)
L-V-8-30 (B)
(a,d) control
(b,e) IgE
(c,f) anti-FcεR (8-30)

Fig. 6

Fig. 7 Strategy for cDNA cloning

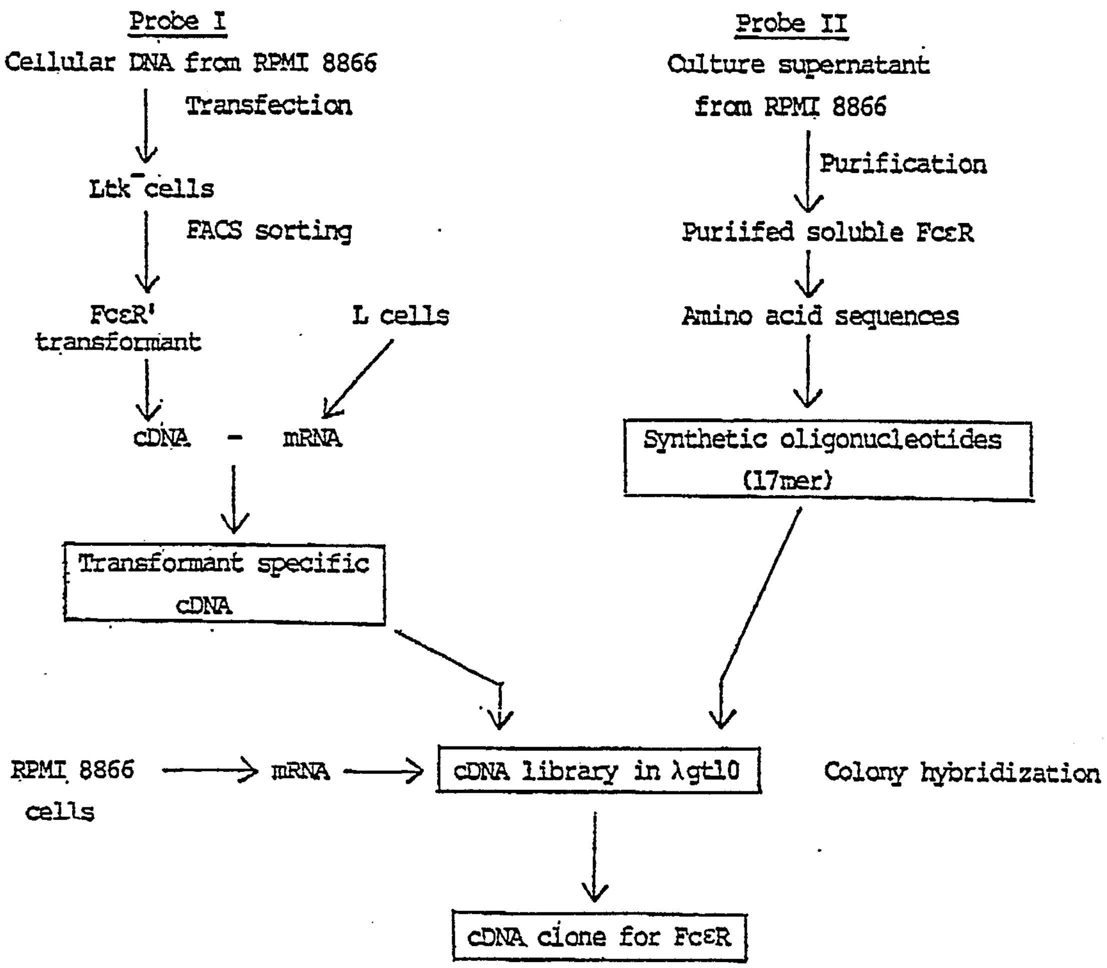

Fig. 8

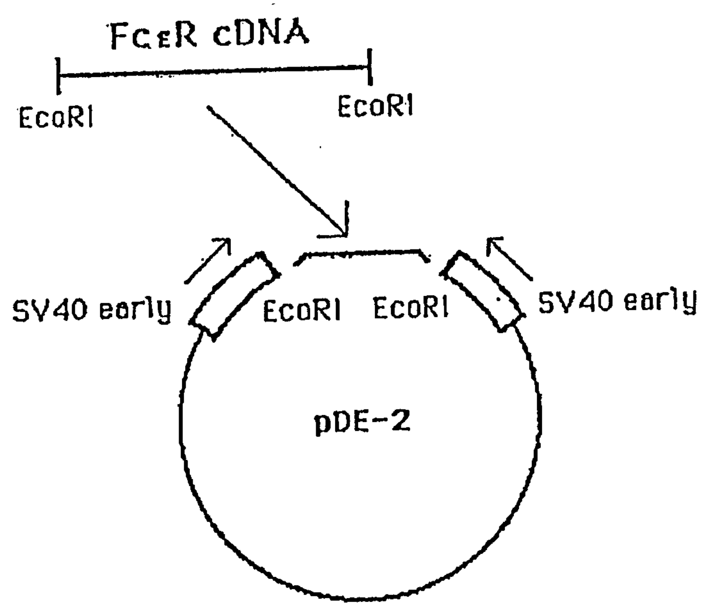
FcεR cDNA
EcoRI
EcoRI
SV40 early
EcoRI
EcoRI
SV40 early
pDE-2

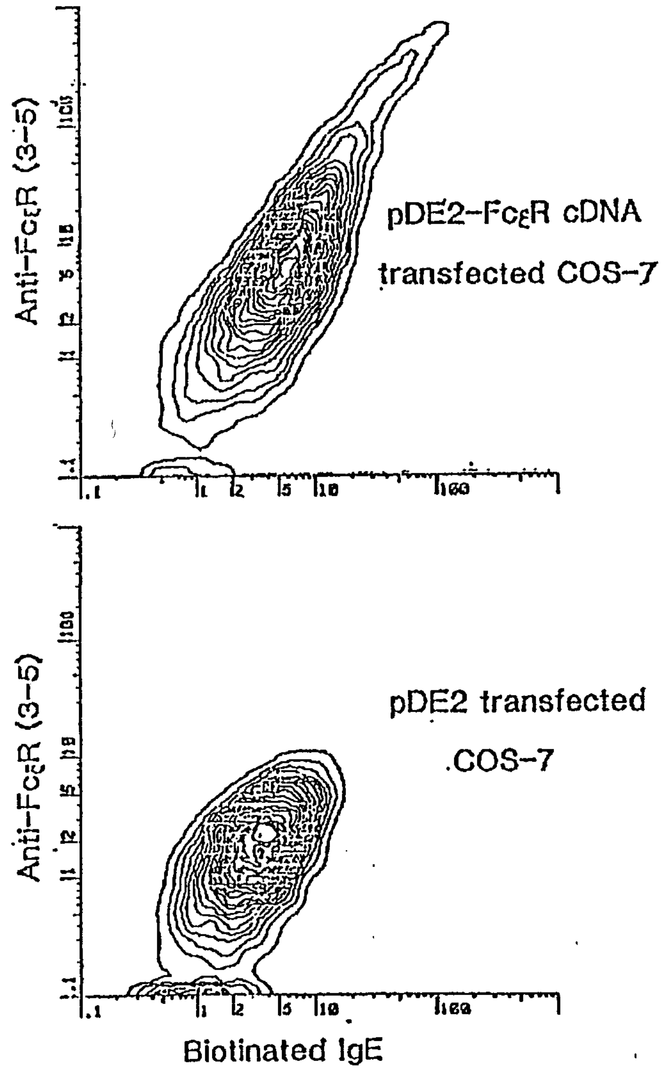
Anti-FcεR (3-5)
pDE2-FcεR cDNA
transfected COS-7
Anti-FcεR (3-5)
pDE2 transfected
COS-7
Biotinated IgE

Fig. 8'

Fig. 9'

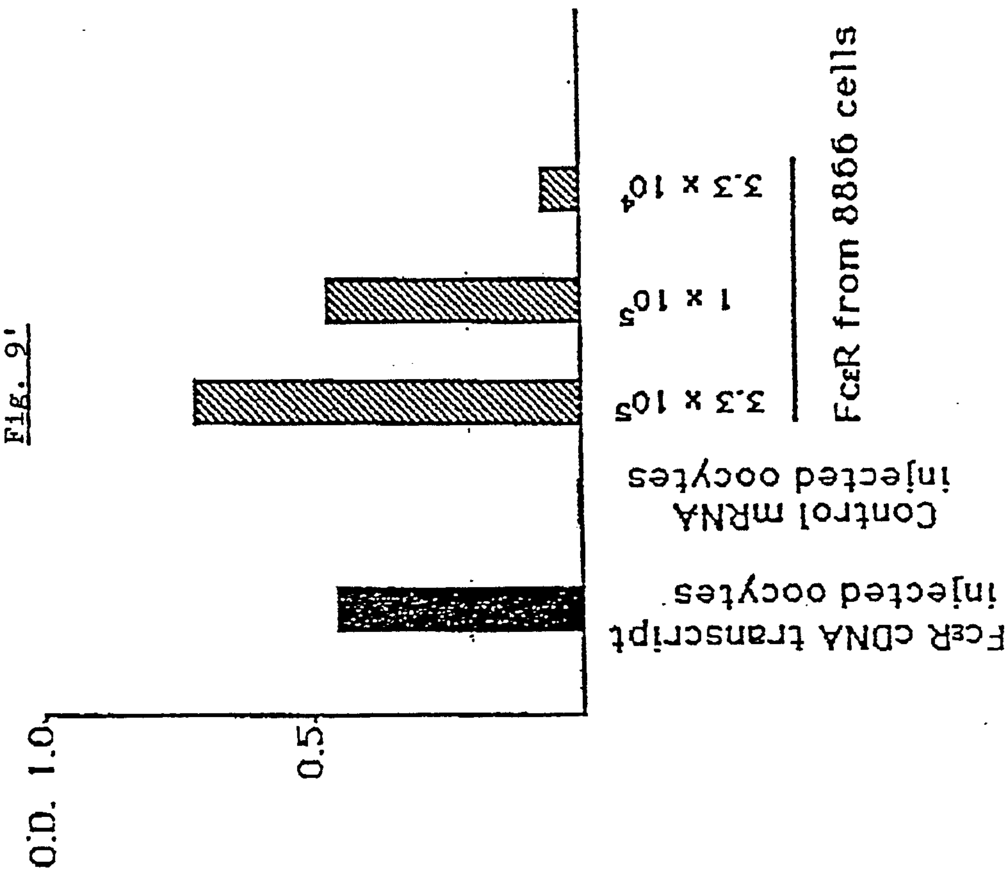
O.D. 1.0
0.5
FcεR cDNA transcript injected oocytes
Control mRNA injected oocytes
3.3 x 10$^5$
1 x 10$^5$
3.3 x 10$^4$
FcεR from 8866 cells

Fig. 9

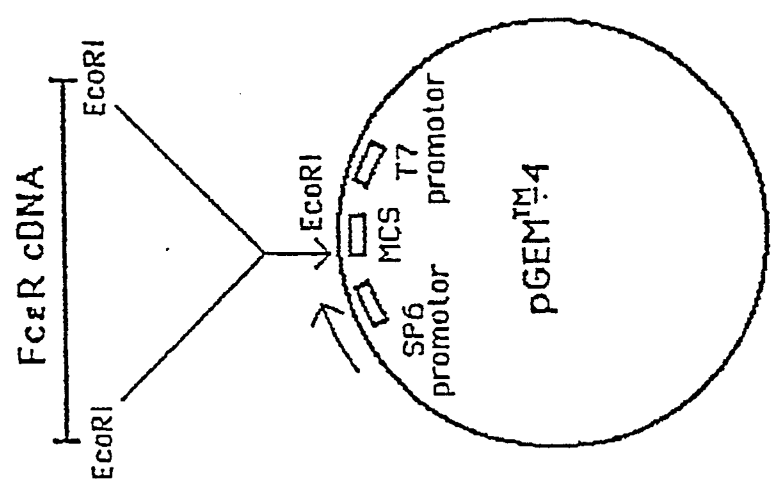
FcεR cDNA
EcoRI
EcoRI
EcoRI
SP6 promotor
MCS
T7 promotor
pGEM™-4

European Patent Office

# EUROPEAN SEARCH REPORT

Application number

EP 86 11 1581

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | THE JOURNAL OF IMMUNOLOGY, vol. 132, no. 2, February 1984, pages 796-803, American Association of Immunologists, US; D.H. CONRAD et al.: "The murine lymphocyte receptor for IgE. I. Isolation and characterization of the murine B cell Fc receptor and comparison with Fc receptors from rat and human" * Page 800, column 1 * --- | 1-4,23 -26 | C 12 N 15/00<br>C 07 K 15/06<br>C 12 P 21/00<br>C 12 Q 1/68<br>A 61 K 37/02 |
| X | THE JOURNAL OF IMMUNOLOGY, vol. 129, no. 2, August 1982, pages 563-569, American Association of Immunologists, US; F.M. MELEWICZ et al.: "Comparison of the Fc receptors for IgE on human lymphocytes and monocytes" * Whole document * --- | 1-4,23 ,25,26 | TECHNICAL FIELDS SEARCHED (Int. Cl.4) |
| A | EUROPEAN JOURNAL OF IMMUNOLOGY, vol. 16, no. 7, July 1986, pages 809-814, VCH Verlagsgesellschaft mbH, Weinheim, DE; T. NAKAJIMA et al.: "IgE receptors on human lymphocytes. I. Identification of the molecules binding to monoclonal anit-Fce receptor antibodies" * Whole document * ----- | 1-4,23 -26 | C 12 N<br>C 12 P |

The present search report has been drawn up for all claims

| Place of search | Date of completion of the search | Examiner |
|---|---|---|
| THE HAGUE | 10-04-1987 | CUPIDO M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82